# EUROPÄISCHE PATENTSCHRIFT

(11) **EP 0 938 569 B1**
(45) Veröffentlichungstag und Bekanntmachung des Hinweises auf die Patenterteilung: **29.10.2008**
(21) Anmeldenummer: 97948804.6
(22) Anmeldetag: 24.10.1997
(51) Int. Cl.: C12N 15/55, C12N 15/82, C12N 1/21, A01H 5/00

(54) **BLATTSPEZIFISCHE EXPRESSION VON GENEN IN TRANSGENEN PFLANZEN**
LEAF-SPECIFIC GENE EXPRESSION IN TRANSGENETIC PLANTS
EXPRESSION DE GENES SPECIFIQUES AUX FEUILLES DANS DES PLANTES TRANSGENIQUES

(30) Priorität: 25.10.1996 DE 19644478
(43) Veröffentlichungstag der Anmeldung: 01.09.1999
(73) Patentinhaber: BASF SE, 67056 Ludwigshafen (DE)
(72) Erfinder: SONNEWALD, Uwe, D-06467 Hoym (DE); EBNETH, Marcus, D-06484 Quedlinburg (DE); SCHMIDT, Ralf-Michael, D-67434 Neustadt (DE)
(74) Vertreter: Kinzebach, Werner
(86) Internationale Anmeldenummer: PCT/EP1997/005900
(87) Internationale Veröffentlichungsnummer: WO 1998/018940

(56) Entgegenhaltungen:
- WO-A-91/05054
- WO-A-93/18169
- WO-A-96/21737
- HARRAK H. ET AL.: "The expression of nuclear genes encoding plastid ribosomal proteins precedes the expression of chloroplast genes during early phases of chloroplast development" PLANT PHYSIOLOGY, Bd. 108, Nr. 2, Juni 1995, Seiten 685-692, XP002058909
- VALDEZ-ALARCON J. ET AL.: "Characterization of a rice sucrose-phosphate synthase-encoding gene" GENE, Bd. 170, Nr. 2, 8.Mai 1996, Seiten 217-222, XP004042829
- ZRENNER R. ET AL.: "Reduction of the cytosolic fructose-1,6-bisphosphatase in transgenic potato plants limits photosynthetic sucrose biosynthesis with no impact on plant growth and tuber yield" THE PLANT JOURNAL, Bd. 9, Nr. 5, Mai 1996, Seiten 671-681, XP002058910

### SEQUENZPROTOKOLL

(1) ALLGEMEINE ANGABE:
   (i) DER:
      (A) NAME: BASF Aktiengesellschaft
      (B) STRASSE: -
      (C) ORT: Ludwigshafen
      (E) LAND: Deutschland
      (F) POSTLEITZAHL: D-67056
   (ii) BEZEICHNUNG DER ERFINDUNG: Blattspezifische Expression von Genen in transgenen Pflanzen
   (iii) ANZAHL DER SEQUENZEN: 5
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: Patent In Release #1.0, Version #1.30 (EPA)
(2) ANGABEN ZU SDQ ID NO: 1:
   (i) SEQUENZKENNZEICHEN:
      (A) LÄNGE: 1720 Basenpaare
      (B) ART: Nucleotid
      (C) STRANGFORM: Doppelstrang
      (D) TOPOLOGIE: linear
   (ii) ART DES MOLEKÜLS: Genom-DNA
   (iii) HYPOTHETISCH: NEIN
   (iv) ANTISENSE: NEIN
   (vi) URSPRÜNLICHE HERKUNFT:
      (A) ORGANISMUS: Solanum tuberosum
      (B) STAMM: Desiree
      (F) GEWEBETYP: Leaf
   (ix) MERKMAL:
      (A) NAME/SCHLÜSSEL: misc_feature
      (B) LAGE:1429..1720
      (D) SONSTIGE ANGABEN:/function= "Teil der Leader-Sequenz der cy-FBPase"
   (ix) MERKMAL:
      (A) NAME/SCHLÜSSEL: promoter
      (B) LAGE:1..1428
   (ix) MERKMAL:
      (A) NAME/SCHLÜSSEL: misc_feature
      (B) LAGE:1429
      (D) SONSTIGE ANGABEN: /function= "Transkriptionsstart"
   (ix) MERKMAL:
      (A) NAME/SCHLÜSSEL: TATA_signal
      (B) LAGE:1399..1405
   (ix) MERKMAL:
      (A) NAME/SCHLÜSSEL: CAAT_signal
      (B) LAGE:1288..1300
   (xi) SEQUENZBESCHREIBUNG: SEQ ID NO: 1:
(2) ANGABEN ZU SEQ ID NO: 2:
   (i) SEQUENZKENNZEICHEN:
      (A) LÄNGE: 1724 Basenpaare
      (B) ART: Nucleotid
      (C) STRANGFORM: Doppelstrang
      (D) TOPOLOGIE: linear
   (ii) ART DES MOLEKÜLS : Genom-DNA
   (iii) HYPOTHETISCH: NEIN
   (iv) ANTISENSE: NEIN
   (vi) URSPRÜNLICHE HERKUNFT:
      (A) ORGANISMUS: Solanum tuberosum
      (B) STAMM: Desiree
      (F) GEWEBETYP: Leaf
   (ix) MERKMAL:
      (A) NAME/SCHLÜSSEL: misc_feature
      (B) LAGE:1433-..1724
      (D) SONSTIGE ANGABEN:/function= "Teil der Leader-Sequenz der cy-FBPase"
   (ix) MERKMAL:
      (A) NAME/SCHLÜSSEL: misc_feature
      (B) LAGE:1..6
      (D) SONSTIGE ANGABEN: /function= "BamHI Restriction Site"
   (xi) SEQUENZBESCHREIBUNG : SEQ ID NO: 2:
   GGATCCAGCT AATGCTGCTC TTGTCACTCA AAATGATGGT ATCCCTCTCG TCATCCAGTG     60
   TTTGTCAAGT CCTGTTAGGA ACACAGTAAG GATATAAACA AACATTTTGT GGTCTTCTTG    120
   GTTATTGAGT GCTTGCTGTT CACTTGTTAA AATTGCACAT ATACGTAGTG AGAAACTCAA    180
   CTGTTGAGTA CCATTGATCC GTCAATCTTG TCGATAACTT TGATAAGGAT ATTTCAGGCA    240
   TCAGACATGT CACCTCTATA GAACTTGGTC TTTTTTTTTA AAAATAAAAA TAAAAATGTT    300
   TGGCATCATA CGAACTTCTG TTACTTTAGG CTGTATCCAG AATAAAATGT TGTTTCCTCA    360
   TTCTGGAATT AGTTGTTTTG CACACGGAAG ACTTTCGAAA TTTACTAATT GTGTTCGTCC    420
   GTCTCAAACT GGCTCACACT TTGGTGGTCA ATTTTACTTC TCAAGGTAAG CAATTACAGA    480
   ATATGAATGT CGCTCTCCTC ATATTTATCC GAACAATAAA AAATGATATC TGTTTGCATA    540
   TGCATGTAGA TCACACACCC CCCCCCCCCC CGCCCCTAGA TTCCCTCGAT TTAGATTAAA    600
   TATAATCATC TACAAGAATT CCGTTGGGCT TCATTATGTG TTTTTACATA TTCGTTTCTG    660
   AACCACCCCC ACCCCGGTGA AAAACATTGC TCTGCCACTG GCTCAATGTA TTGACACAAA    720
   TGAACTTCAA ACTGGGCAGG TGAATTATGC TCTAGGAGCA TTGTATTATC TATGCAATGC    780
   ATCAAACAAG GAAGAGATCT TAAAGCCAGA AGTAATTGAT GCAATCAAAA GTTATGCAGC    840
   TGCAGGTGGA GTTAGTACAA GCTTCAGTAA TTTGGCTCAG GCTTTCTTAG ATCAACATGT    900
   TCCTCAGCTT AATTAAAATG GAGGAAACCA AAGATTATGT TGTAAAATCA TTTTCTATCC    960
   TAGATGGTCT ATCGGAAACA ATTTATTTAT TACTCCTATC CAATTCATTA TATTTTCAAA   1020
   AGTTATGAAG TCCACGAAAT ATGTGACGTG GGTAAAGAAG ACCCATGCCA AGCCAGTGGG   1080
   ATATAGAAAC AAAACATGTA ATAAAGAGAA CAAATAATGA GTTTCGAAAA GAACAGAAGT   1140
   TAGCATAAGG ACGAGAATCA CATTATCTTA GGTGCCAACC ACTAATCCTA TGTATCATTC   1200
   TCCTCTTTCC ACGTGTCATC CTACACTTCC TTTGCCATCA GATTAGATAG CCCGGTTAGT   1260
   ACCTACACTG TATATCAAAA AATACGTAAC AATCATCCAA ACATATCATC GATCAAAGGA   1320
   TATTTATCTT GATGTGCTTT CGCCGTCCAT TGTAACGAGT TTGGATGAAT TTGATATACA   1380
   CCCACTCAGA TATCAATATA TTTTATAAAA AGAAACAAAA TTGAATACTA GTAATATCTA   1440
   TGTAGATATT TATTTTTTCA ACAATCCTGT AAGTTATAAG GATAACTCAC TTATATGTGA 1500
   CGTGGATAAT GAAGAGCTAG GCAGGCAGTG AGAGATAGAA ACAAATTAAG CAGAGACGAA 1560
   AAACAAATCA GTTAACAGAA TGACGAATTG GATCACGCTT TATCTTAGTG CCAACCACTG 1620
   ATCCCATGCA TCACTCTGCT CTTTCCACGT GGCATCCTCT GACGTCAGAT CAGATTCCTC 1680
   TTCTTTCTTT TTTTTTTCTG TATATATATG AGCATTTTAG TAGT 1724
(2) ANGABEN ZU SEQ ID NO: 3:
   (i) SEQUENZKENNZEICHEN:
      (A) LÄNGE: 1109 Basenpaare
      (B) ART: Nucleotid
      (C) STRANGFORM: Doppelstrang
      (D) TOPOLOGIE: linear
   (ii) ART DES MOLEKÜLS: Genom-DNA
   (iii) HYPOTHETISCH: NEIN
   (iv) ANTISENSE: NEIN
   (vi) URSPRÜNLICHE HERKUNFT:
      (A) ORGANISMUS: Solanum tuberosum
      (B) STAMM: Desiree
      (F) GEWEBETYP: Leaf
   (ix) MERKMAL:
      (A) NAME/SCHLÜSSEL: misc_feature
      (B) LAGE: 1..6
      (D) SONSTIGE ANGABEN: /function= "EcoRI Restriction Site"
   (xi) SEQUENZBESCHREIBUNG: SEQ ID NO: 3:
   GAATTCCGTT GGGCTTCATT ATGTGTTTTT ACATATTCGT TTCTGAACCA CCCCCACCCC      60
   GGTGAAAAAC ATTGCTCTGC CACTGGCTCA ATGTATTGAC ACAAATGAAC TTCAAACTGG     120
   GCAGGTGAAT TATGCTCTAG GAGCATTGTA TTATCTATGC AATGCATCAA ACAAGGAAGA     180
   GATCTTAAAG CCAGAAGTAA TTGATGCAAT CAAAAGTTAT GCAGCTGCAG GTGGAGTTAG     240
   TACAAGCTTC AGTAATTTGG CTCAGGCTTT CTTAGATCAA CATGTTCCTC AGCTTAATTA     300
   AAATGGAGGA AACCAAAGAT TATGTTGTAA AATCATTTTC TATCCTAGAT GGTCTATCGG     360
   AAACAATTTA TTTATTACTC CTATCCAATT CATTATATTT TCAAAAGTTA TGAAGTCCAC     420
   GAAATATGTG ACGTGGGTAA AGAAGACCCA TGCCAAGCCA GTGGGATATA GAAACAAAAC     480
   ATGTAATAAA GAGAACAAAT AATGAGTTTC GAAAAGAACA GAAGTTAGCA TAAGGACGAG     540
   AATCACATTA TCTTAGGTGC CAACCACTAA TCCTATGTAT CATTCTCCTC TTTCCACGTG     600
   TCATCCTACA CTTCCTTTGC CATCAGATTA GATAGCCCGG TTAGTACCTA CACTGTATAT     660
   CAAAAAATAC GTAACAATCA TCCAAACATA TCATCGATCA AAGGATATTT ATCTTGATGT     720
   GCTTTCGCCG TCCATTGTAA CGAGTTTGGA TGAATTTGAT ATACACCCAC TCAGATATCA     780
   ATATATTTTA TAAAAAGAAA CAAAATTGAA TACTAGTAAT ATCTATGTAG ATATTTATTT     840
   TTTCAACAAT CCTGTAAGTT ATAAGGATAA CTCACTTATA TGTGACGTGG ATAATGAAGA     900
   GCTAGGCAGG CAGTGAGAGA TAGAAACAAA TTAAGCAGAG ACGAAAAACA AATCAGTTAA     960
   CAGAATGACG AATTGGATCA CGCTTTATCT TAGTGCCAAC CACTGATCCC ATGCATCACT    1020
   CTGCTCTTTC CACGTGGCAT CCTCTGACGT CAGATCAGAT TCCTCTTCTT TCTTTTTTTT    1080
   TTCTGTATAT ATATGAGCAT TTTAGTAGT                                      1109
(2) ANGABEN ZU SEQ ID NO: 4:
   (i) SEQUENZKENNZEICHEN:
      (A) LÄNGE: 1428 Basenpaare
      (B) ART: Nucleotid
      (C) STRANGFORM: Doppelstrang
      (D) TOPOLOGIE: linear
   (ii) ART DES MOLEKÜLS: Genom-DNA
   (iii) HYPOTHETISCH: NEIN
   (iv) ANTISENSE: NEIN
   (vi) URSPRÜNLICHE HERKUNFT:
      (A) ORGANISMUS: Solanum tuberosum
      (B) STAMM: Desiree
      (F) GEWEBETYP: Leaf
   (xi) SEQUENZBESCHREIBUNG: SEQ ID NO: 4:
   CCAGCTAATG CTGCTCTTGT CACTCAAAAT GATGGTATCC CTCTCGTCAT CCAGTGTTTG    60
   TCAAGTCCTG TTAGGAACAC AGTAAGGATA TAAACAAACA TTTTGTGGTC TTCTTGGTTA   120
   TTGAGTGCTT GCTGTTCACT TGTTAAAATT GCACATATAC GTAGTGAGAA ACTCAACTGT   180
   TGAGTACCAT TGATCCGTCA ATCTTGTCGA TAACTTTGAT AAGGATATTT CAGGCATCAG   240
   ACATGTCACC TCTATAGAAC TTGGTCTTTT TTTTTAAAAA TAAAAATAAA AATGTTTGGC   300
   ATCATACGAA CTTCTGTTAC TTTAGGCTGT ATCCAGAATA AAATGTTGTT TCCTCATTCT   360
   GGAATTAGTT GTTTTGCACA CGGAAGACTT TCGAAATTTA CTAATTGTGT TCGTCCGTCT   420
   CAAACTGGCT CACACTTTGG TGGTCAATTT TACTTCTCAA GGTAAGCAAT TACAGAATAT   480
   GAATGTCGCT CTCCTCATAT TTATCCGAAC AATAAAAAAT GATATCTGTT TGCATATGCA   540
   TGTAGATCAC ACACCCCCCC CCCCCCCGCC CCTAGATTCC CTCGATTTAG ATTAAATATA   600
   ATCATCTACA AGAATTCCGT TGGGCTTCAT TATGTGTTTT TACATATTCG TTTCTGAACC   660
   ACCCCCACCC CGGTGAAAAA CATTGCTCTG CCACTGGCTC AATGTATTGA CACAAATGAA   720
   CTTCAAACTG GGCAGGTGAA TTATGCTCTA GGAGCATTGT ATTATCTATG CAATGCATCA   780
   AACAAGGAAG AGATCTTAAA GCCAGAAGTA ATTGATGCAA TCAAAAGTTA TGCAGCTGCA   840
   GGTGGAGTTA GTACAAGCTT CAGTAATTTG GCTCAGGCTT TCTTAGATCA ACATGTTCCT   900
   CAGCTTAATT AAAATGGAGG AAACCAAAGA TTATGTTGTA AAATCATTTT CTATCCTAGA   960
   TGGTCTATCG GAAACAATTT ATTTATTACT CCTATCCAAT TCATTATATT TTCAAAAGTT  1020
   ATGAAGTCCA CGAAATATGT GACGTGGGTA AAGAAGACCC ATGCCAAGCC AGTGGGATAT  1080
   AGAAACAAAA CATGTAATAA AGAGAACAAA TAATGAGTTT CGAAAAGAAC AGAAGTTAGC  1140
   ATAAGGACGA GAATCACATT ATCTTAGGTG CCAACCACTA ATCCTATGTA TCATTCTCCT  1200
   CTTTCCACGT GTCATCCTAC ACTTCCTTTG CCATCAGATT AGATAGCCCG GTTAGTACCT  1260
   ACACTGTATA TCAAAAAATA CGTAACAATC ATCCAAACAT ATCATCGATC AAAGGATATT  1320
   TATCTTGATG TGCTTTCGCC GTCCATTGTA ACGAGTTTGG ATGAATTTGA TATACACCCA  1380
   CTCAGATATC AATATATTTT ATAAAAAGAA ACAAAATTGA ATACTAGT                1428
(2) ANGABEN ZU SEQ ID NO: 5:
   (i) SEQUENZKENNZEICHEN:
      (A) LÄNGE: 817 Basenpaare
      (B) ART: Nucleotid
      (C) STRANGFORM: Doppelstrang
      (D) TOPOLOGIE: linear
   (ii) ART DES MOLEKÜLS: Genom-DNA
   (iii) HYPOTHETISCH: NEIN
   (iv) ANTISENSE: NEIN
   (vi) URSPRÜNLICHE HERKUNFT:
      (A) ORGANISMUS: Solanum tuberosum
      (B) STAMM: Desiree
      (F) GEWEBETYP: Leaf
   (ix) MERKMAL:
      (A) NAME/SCHLÜSSEL: misc_feature
      (B) LAGE:1..6
      (D) SONSTIGE ANGABEN: /function= "EcoRI Restriction Site"
   (xi) SEQUENZBESCHREIBUNG: SEQ ID NO: 5:
   GAATTCCGTT GGGCTTCATT ATGTGTTTTT ACATATTCGT TTCTGAACCA CGCCCACCCC    60
   GGTGAAAAAC ATTGCTCTGC CACTGGCTCA ATGTATTGAC ACAAATGAAC TTCAAACTGG   120
   GCAGGTGAAT TATGCTCTAG GAGCATTGTA TTATCTATGC AATGCATCAA ACAAGGAAGA   180
   GATCTTAAAG CCAGAAGTAA TTGATGCAAT CAAAAGTTAT GCAGCTGCAG GTGGAGTTAG   240
   TACAAGCTTC AGTAATTTGG CTCAGGCTTT CTTAGATCAA CATGTTCCTC AGCTTAATTA   300
   AAATGGAGGA AACCAAAGAT TATGTTGTAA AATCATTTTC TATCCTAGAT GGTCTATCGG   360
   AAACAATTTA TTTATTACTC CTATCCAATT CATTATATTT TCAAAAGTTA TGAAGTCCAC   420
   GAAATATGTG ACGTGGGTAA AGAAGACCCA TGCCAAGCCA GTGGGATATA GAAACAAAAC   480
   ATGTAATAAA GAGAACAAAT AATGAGTTTC GAAAAGAACA GAAGTTAGCA TAAGGACGAG   540
   AATCACATTA TCTTAGGTGC CAACCACTAA TCCTATGTAT CATTCTCCTC TTTCCACGTG   600
   TCATCCTACA CTTCCTTTGC CATCAGATTA GATAGCCCGG TTAGTACCTA CACTGTATAT   660
   CAAAAAATAC GTAACAATCA TCCAAACATA TCATCGATCA AAGGATATTT ATCTTGATGT   720
   GCTTTCGCCG TCCATTGTAA CGAGTTTGGA TGAATTTGAT ATACACCCAC TCAGATATCA   780
   ATATATTTTA TAAAAAGAAA CAAAATTGAA TACTAGT                             817

## Beschreibung

Die vorliegende Erfindung betrifft Nucleinsäure-Sequenzen mit Promotoraktivität, die im wesentlichen eine Promotorsequenz einer cytosolischen Fructose -1,6-bisphosphatase von Pflanzen der Gattung Solanum umfassen und in Pflanzen eine mesophyllspezifische Expression von ihnen kontrollierter codierender Nucleotid-Sequenzen bewirken, Expressionskassetten, rekombinante Vektoren und Mikroorganismen, die solche regulativen Sequenzen umfassen, damit transformierte transgene Pflanzen, ein Verfahren zur Herstellung transgener Pflanzen sowie ein Verfahren zur Isolierung des blattspezifischen Promotors.

Es ist bekannt, mittels gentechnischer Verfahren gezielt Fremdgene in das Genom einer Pflanze zu übertragen. Dieser Prozeß wird als Transformation und die resultierenden Pflanzen werden als transgen bezeichnet. Transgene Pflanzen werden derzeit in unterschiedlichen biotechnologischen Bereichen eingesetzt. Die vornehmlichen Ziele sind zum einen der Pflanzenschutz und zum anderen eine Qualitätssteigerung der erntbaren Produkte. Zur wirksamen Expression von Fremdgenen in Pflanzen sind Regulationssignale notwendig, die eine ordnungsgemäße Transkription ermöglichen. Hierzu gehören Promotoren und Terminatoren. Die am 3'-Ende der kodierenden DNA befindlichen Terminatoren dienen der Beendigung der Transkription und gegebenenfalls als Signal zur Polyadenylierung der gebildeten mRNA. Promotoren enthalten Erkennungssequenzen für RNA-Polymerasen und für transkriptionale Effektoren. Die Promotoren sind für das Expressionsverhalten der Fremdgene verantwortlich.

Herbizidtolerante Pflanzen, wie sie aus der DE-A-3701623 bekannt sind, stellen ein Beispiel für gentechnische Pflanzenschutzmaßnahmen dar. Weitere Beispiele sind insektenresistente Pflanzen (Vaek et al. (1987) Plant Cell 5, 159-169), virusresistente Pflanzen (Powell et al. (1986) Science 232, 738-743) und ozonresistente Pflanzen (Van Camp et al. (1994) BioTech. 12, 165-168). Beispiele für gentechnisch erzielte Qualitätssteigerungen sind: Erhöhung der Haltbarkeit von Früchten (Oeller et al. (1991) Science 254, 437-439), Erhöhung der Stärkeproduktion in Kartoffelknollen (Stark et al. (1992) Science 242, 419), Veränderung der Stärke- (Visser et al. (1991) Mol. Gen. Genet. 225, 289-296) und Lipidzusammensetzung (Voelker et al. (1992) Science 257, 72-74) und Produktion pflanzenfremder Polymere (Poirer et al. (1992) Science 256, 520-523).

Eine Vielzahl von Promotoren, die die Expression von Fremdgenen in Pflanzen steuern, ist bekannt. Der am häufigsten verwendete Promotor ist der 35S CaMV-Promotor (Franck et al. (1980) Cell 21, 285-294). Dieser Promotor enthält unterschiedliche Erkennungssequenzen für transkriptionale Effektoren, die in ihrer Gesamtheit zu einer konstitutiven Expression des eingeführten Gens führen (Benfey et al. (1989) EMBO J. 8, 2195-2202). Häufig werden auch induzierbare oder zell- oder gewebespezifische Promotoren eingesetzt.

Unter anderem wurden folgende Beispiele für eine induzierbare Expression beschrieben: Wundinduktion (DE-A-3843628, DE-B-3837752), chemische Induktion (Ward et al. (1993) Plant Molec. Biol. 22, 361-366) und Lichtinduktion (Fluhr et al. (1986) Science 232, 1106-1112).

Aus der DE-A-4207358 ist ein Promotor bekannt, der eine Schließzellen-spezifische Genexpression jedoch keine spezifische Expression in Mesophyllzellen oder epidermalen Zellen von Blättern bewirkt. Durch künstliche Veränderung der Öffnungsperioden der Stomata kann der Gasaustausch entsprechend manipulierter Pflanzen wunschgemäß reguliert werden. Herbizidtoleranz oder -resistenz kann durch einen solchen Promotor nicht vermittelt werden.

Weitere Beispiele für zell- und gewebespezifische Expression sind: samen-, knollen- und fruchtspezifische (zusammengefaßt in Edwards und Coruzzi (1990) Annu. Rev. Genet. 24, 275-303; DE-A-3843627), phloemspezifische (Schmülling et al. (1989) Plant Cell 1, 665-670), wurzelknöllchenspezifische (DE-A-3702497) und meristemspezifische (Ito et al. (1994) Plant Molec. Biol. 24, 863-878) Expression. Beispiele für Promotoren in chloroplastenhaltigen Zellen sind gleichfalls aus Edwards und Coruzzi (1990), Annu. Rev. Genet. 24, 277-279 bekannt. Die darin beschriebenen Promotoren bewirken die Expression entweder nur in induzierbarer Form (z.B. der rbcS-3A-Promotor) oder nur in bestimmten Zelltypen (z.B. die GS2- und GS3A-Promotoren), jedoch ist die Expression nicht auf bestimmte Pflanzenteile beschränkt.

Die Verwendung der beschriebenen Promotoren ist oft problematisch. Promotoren, die eine konstitutive Expression der von ihnen kontrollierten Gene bewirken, können beispielsweise zur Erzeugung herbizidtoleranter und pathogenresistenter Pflanzen eingesetzt werden, haben aber den Nachteil, daß die Produkte der von ihnen kontrollierten Gene in allen Teilen der Pflanze vorliegen, auch in den geernteten Pflanzenteilen, was in manchen Fällen unerwünscht sein kann. Induzierbare Promotoren sind gleichfalls nicht unproblematisch, da die Induktionsbedingungen bei landwirtschaftlich genutzten Pflanzen im Freiland typischerweise schwer kontrollierbar sind.

In C3-Pflanzen führt der aus einer C4-Pflanze stammende Promotor der Phosphoenolpyruvat Carboxylase zu Expression im Mesophyll des Blattes (Stockhaus et al., (1994), Mol. Gen. Genet. 245, 286-293). Dieser Promotor vermittelt im Mesophyll jedoch nur niedrige Aktivität. Außerdem zeigt er in Wurzeln ebenfalls Aktivität. Diese geringe Organspezifität ist für viele Anwendungen unerwünscht.

Promotoren, die eine mesophyllspezifische, vorzugsweise permanente Expression von Genen, die von ihnen kontrolliert werden, bewirken, sind nicht bekannt.

Zrenner et al. beschrieben die Klonierung eines Fructose-1,6-bisphosphatasegens aus Blättern der Kartoffel (Zrenner et al. (1996), The Plant Journal 9(5), 671-681).

Die WO 93/18169 offenbart Transkription regulierende DNA-Sequenzen, die zu Expression in Schließzellen führen, nicht aber in Mesophyllzellen.

Harrak et al. erwähnen einen Promotor der Spinatpflanze, der Transkription in photosynthetischen Geweben bewirkt, aber eine Induktion erfordert (Harrak et al. (1995), Plant Physiol. 108, 685-692). Die WO 91/05054 offenbart einen aus Weizen isolierten Promotor, der ebenfalls eine Induktion (Licht) erfordert. Valdez-Alarcon et al. beschreiben ein für Sacharose-Phosphat-Synthase codierendes Gen der Reispflanze (Valdez-Alarcon et al. (1996), Gene 170, 217-222). Das Gen wird offenbar entwicklungsabhängig vorwiegend in Blättern transkribiert.

Es wäre deshalb wünschenswert, Wege zu finden, Gene unter Umgehung dieser Nachteile in Pflanzen mesophyllspezifisch zu exprimieren.

Der Erfindung liegt deshalb die Aufgabe zugrunde, Mittel bereitzustellen, die eine gezielte mesophyllspezifische Genexpression in Pflanzen ermöglichen. Diese Mittel sollten beispielsweise zur Expression von Resistenzgenen und die Photosyntheleistung modifizierenden Genen geeignet sein.

Diese Aufgabe wurde überraschenderweise gelöst durch Bereitstellung eines neuen Promotors gemäß der Definition des Hauptanspruchs, der in Pflanzen eine, vorzugsweise permanente, blattspezifische Expression einer von ihm kontrollierten codierenden Nucleotid-Sequenz unabhängig von Induktionsfaktoren bewirkt.

Die Erfindung wird nun unter Bezugnahme auf die folgenden Figuren näher erläutert. Dabei zeigt
- Figur 1 (A): eine schematische Darstellung des in den Vektor pUC19 klonierten ca. 7100 bp umfassenden BamHI-Fragmentes des Klons FBP-1 aus Kartoffel. Der cytosolische FBPase(cy-FBPase)-Promotorbereich ist schwarz hervorgehoben; (B) das Konstruktionsschema von Plasmid FBP:pBlue;
- Figur 2: die Nucleotidsequenz des cy-FBPase-Promotors aus Kartoffel. Der zum 3'-Ende des für die Southernhydridisierung verwendeten 5'-Subfragmentes der cy-FBPase komplementäre Bereich ist unterstrichen; zwei palindromische Sequenzabschnitte sind punktiert unterstrichen; die 5'-terminale Sequenz "GGATC" wurde zur Erzeugung einer BamHI-Schnittstelle an die genomische DNA angefügt;
- Figur 3 (A): das Konstruktionsschema der Plasmide FBP:GUS und FBP:GUS(DEL); (B) eine schematische Darstellung des Plasmids FBP:GUS, mit dem ca. 1700 bp umfassenden FBPase-Promotor, dem ca. 1870 bp umfassenden GUS-Gen und dem ca. 260 bp umfassenden Nopalin-Synthase Terminator, insertiert in Vektor pBI 101;
- Figur 4: ein Balkendiagramm, das die durch den cy-FBPase-Promotor kontrollierte blattspezifische GUS-Aktivität in transgenen Kartoffelpflanzen belegt; es werden die für zwei verschiedene Transformationsexperimente mit FBP:GUS ermittelten Resultate (Pflanzenlinie "Me 1-22" und "Me 1-9") gezeigt und mit einem Kontrollversuch ("Kontrolle") verglichen;
- Figur 5: ein Balkendiagramm, das die durch den cy-FBPase-Promotor kontrollierte, blattspezifische GUS-Aktivität in transformierten Tabakpflanzen belegt. "TME-1/67" bezeichnet die mit dem Vektor FBP:GUS erzielten Resultate. "TME-11/13" bezeichnet die mit dem Vektor FBP:GUS(DEL) erzielten Resultate. "WT" zeigt die für den Wildtyp ermittelten Resultate. Angegeben ist die Menge gebildetes 4-Methyl-umbelliferon pro Milligramm Protein, pro Minute;
- Figur 6: den histochemischen Nachweis der GUS-Aktivität in verschiedenen Geweben des Tabakblattes einer transgenen Pflanze. (A) Querschnitt durch das zentrale Leitgewebe des Source-Blattes, (B) Epidermis, (C) Querschnitt durch die Petiole, (D) Querschnitt durch das Mesophyll eines Source-Blattes. Die Schnitte wurden 20 min in 3%iger Paraformaldehydlösung fixiert und anschließend über Nacht in X-Gluc-Lösung inkubiert. Danach wurde das Chlorophyll durch 70%iges Ethanol entfernt;
- Figur 7: einen Northern-blot, der die gleichmäßige blattspezifische GUS-Expression in transgenen Tabakpflanzen, vermittelt durch den cy-FBPase-Promotor aus Kartoffel, belegt;
- Figur 8: einen histochemischen Nachweis der β-Glucuronidase(GUS)-Aktivität in Tabakkeimlingen;
- Figur 9: eine schematische Darstellung des Plasmides pBin-FBP, mit dem 1724 bp umfassenden cy-FBPase-Promotor aus Kartoffel und dem 280 bp umfassenden Octopin-Synthase Terminator, insertiert in Vektor pBin 19; und
- Figur 10: cDNA-Sonde des cy-FBPase Gens aus Kartoffel (EMBL-Nr.: X76946).

Der Begriff "Gen" bzw. "codierende (Nucleotid-) Sequenz" bezeichnet im Rahmen der vorliegenden Erfindung eine Nucleotid-Sequenz, die eine bestimmte, gegebenenfalls vererbbare, Struktur, beispielsweise wenigstens ein Protein, wenigstens ein Ribozym oder wenigstens eine Antisense-RNA; oder Funktion, wie beispielsweise Resistenz, codiert; oder eine Änderung der Zusammensetzung pflanzlicher Inhaltsstoffe, wie Öle, Fette, Enzyme, Proteine, Biopolymere, bewirkt, so dass z. B. der Nährwert, der Ernteertrag oder die industrielle Nutzbarkeit der Pflanze verbessert wird.

Ein "Promotor" bezeichnet erfindungsgemäß einen Nucleotidsequenzbereich, welcher die Transkription eines Gens, bzw. die Synthese der entsprechenden mRNA steuert. Der Promotor umfasst eine Sequenz, die 5'-stromaufwärts vom Transkriptionsstartpunkt liegt. Sie umfasst als wesentliches Sequenzelement wenigstens die sogenannte "TATA"-Box. Weitere regulative Elemente, wie die "CAAT"-Box oder eine GC-Box können ebenfalls enthalten sein. Darüber hinaus kann es erforderlich sein, dass die erfindungsgemäße Promotorsequenz zusätzlich zu dem oben genannten Sequenzabschnitt eine 3'-stromabwärts vom Transkriptionsstartpunkt gelegene Sequenz, wie z. B. eine Leadersequenz oder einen Teilbereich davon aufweist, um die gewünschte Promotor-Aktivität und/oder -Spezifität zu zeigen oder voll zu entfalten.

"Resistenz" bedeutet im Rahmen der vorliegenden Erfindung die künstlich induzierte Resistenz oder Toleranz der transgenen Pflanzen gegen Herbizide und/oder Pathogene, wie z.B. Pilze, Viren oder Insekten, gegen bestimmte äußere Bedingungen, wie hohe Konzentrationen an Ozon, Schwefeldioxid, Stickoxiden oder anderen exogenen Schadstoffen sowie gegen Hitze, Kälte, Trockenheit oder UV-Licht.

Eine "Modifikation der Photosyntheseleistung einer Pflanze" umfaßt die Verringerung und insbesondere die Erhöhung der photosynthetischen Aktivität der transformierten Pflanzen. Dies kann beispielsweise dadurch erfolgen, daß man Gene exprimiert, welche gezielt die Lichtausbeute der Pflanze erhöhen, die Umsatzgeschwindigkeit einzelner geschwindigkeitsbestimmender Stoffwechselschritte erhöhen, oder den Stoffaustausch mit der Umgebung beeinflussen.

"Blattspezifität" bedeutet im Rahmen der vorliegenden Erfindung, daß ein unter der Kontrolle eines erfindungsgemäßen Promotor stehendes Fremdgen im gesamten Blattorgan oder in.bestimmten Geweben des Blattes, vorzugsweise im Mesophyll (z.B. Palisadenparenchym) exprimiert wird, nicht aber im Sproß oder in anderen Pflanzenteilen wie insbesondere den Wurzeln. Insbesondere ist "Blattspezifität" im Rahmen der vorliegenden Erfindung auch dann gegeben, wenn der erfindungsgemäße Promotor die Expression eines Fremdgens im Blatt, vorzugsweise im Mesophyll von Blättern, insbesondere von Source-Blättern, im Vergleich zu anderen Pflanzenteilen, wie Stamm, nichtkeimenden Knollen, Früchten oder Samen der Pflanze begünstigt und in Blättern eine signifikant, wie z. B. mindestens etwa 5 bis 10-fach, wie etwa 10- bis 100-fach, erhöhte Expression bewirkt,

"Source-Blätter" einer Pflanze sind die älteren Blätter einer Pflanze, welche im Überschuss Kohlenstoff durch Photosynthese fixieren und somit gebundenen Kohlenstoff in andere Pflanzenteile, wie z. B. die jüngeren "Sink"-Blätter, exportieren.

Eine "permanente" Expression bedeutet im Rahmen der vorliegenden Erfindung die von exogen applizierten chemischen Induktionssignalen im wesentlichen unabhängige über eine oder mehrere Pflanzengenerationen andauernde Expression des unter der Kontrolle des erfindungsgemäßen Promotors stehenden Gens.

Ein erster Gegenstand der vorliegenden Erfindung betrifft Promotoren gemäß der Definition des Hauptanspruchs, die in Pflanzen eine, vorzugsweise permanente, mesophyllspezifische Expression von ihnen kontrollierter kodierender Nucleotid-Sequenzen bewirken.

Der primäre Wirkort von Herbiziden und einer Vielzahl von Pathogenen ist das Blattgewebe, so daß eine blattspezifische Expression der entsprechenden Resistenzgene ausreichenden Schutz bieten würde. Da die Photosynthese gleichfalls im Blattgewebe abläuft, wäre zur Modifikation und insbesondere zur Verbesserung der photosynthetischen Leistungsfähigkeit die blattspezifische Expression eines oder mehrerer, die Photosyntheseleistung beeinflussender Gene notwendig.

Die erfindungsgemäßen Promotoren bieten nun den überraschenden Vorteil, Resistenzgene spezifisch am eigentlichen Wirkort in der Pflanze exprimieren zu können. Andererseits ist die gezielte Beeinflussung der Photosyntheseleistung mit den erfindungsgemäßen Promotoren erstmals möglich. Wie die Versuchsergebnisse überraschenderweise belegen, ermöglichen bevorzugte Promotoren erstmals die spezifische Lokalisierung und Expression eines Fremdgens im Mesophyll von Blättern, insbesondere von Source-Blättern, während im parenchymatischen Gewebe, sowie Xylem, Phloem und anderen keine Aktivität zu beobachten ist.

Ein erfindungsgemäßer Promotor kann durch Isolierung und Charakterisierung von Promotoren blattspezifisch und vorzugsweise permanent exprimierter Gene bereitgestellt werden. Bevorzugt sind Promotoren, die im wesentlichen den Promotoren der cytosolischen Fructose-1,6-Bisphosphatase-Gene (cy-FBPase-Gene) aus blattspezifischen Mesophyllzellen von Pflanzen entsprechen. Besonders bevorzugt sind erfindungsgemäß cy-FBPase Promotoren, die aus blattspezifischen Mesophyllzellen von Pflanzen der Gattung Solanum (Kartoffel) isoliert wurden, sowie funktionelle Äquivalente davon. Eine bevorzugte Ausführungsform betrifft eine Nucleotid-Sequenz mit der gewünschten Promotoraktivität, die aus Solanum tuberosum var. Desiree isoliert wird, oder funktionelle Äquivalente davon. Insbesondere bevorzugt ist ein Promotor mit einer Nucleotidsequenz, ausgewählt unter SEQ ID NO: 1 bis 5, oder funktionellen Äquivalenten dieser Sequenzen.

Der Transkriptionsstart in der bevorzugten Nucleotidsequenz gemäß SEQ ID NO: 1 wurde mit Hilfe von "Primer Extension" am A.L.F. (Automatic Laser Fluorescence DNA Sequenzer (Pharmacia)) festgelegt. Dazu wurde ein 5'-fluoreszenzmarkiertes Oligonukleotid angefertigt, das zu einer 21 bp langen Region im Promotor von +1577 bis +1599 (SEQ ID NO: 1) komplementär ist. Mit Hilfe dieses Primers wurde Gesamt-RNA aus Source-Blättern in einzelsträngige cDNA umgeschrieben. Die nicht durch den Primer erkannte RNA sowie die RNA-Anteile der cDNA/RNA-Hybride wurden anschließend verdaut. Die cDNA wurde dann am A.L.F. gleichzeitig mit der mit dem gleichen Primer sequenzierten Promotor DNA analysiert. Der Transkriptionsstart konnte durch Vergleich der Signale im Sequenzgel identifiziert werden. Die Sequenz SEQ ID NO: 1 umfasst demnach 1428 bp Promotorregion (SEQ ID NO: 4) und 292 bp 5'-untranslatierten Bereich der cyt FBPase. 30 bp oberhalb des Startpunktes konnte eine TATA-Box-Sequenz gefunden werden ("TTATAAA"), sowie 141 bp oberhalb des Startpunktes eine CAAT-Box ("ATCATCCAAACAT"). Außerdem wurden mehrere invertierte und direkte Sequenzwiederholungen festgestellt, die keine Homologien zu Sequenzwiederholungen aufweisen, die in anderen Promotoren gefunden wurden.

Die ermittelten direkten und invertierten Sequenzwiederholungen mit einer Länge von mindestens 10bp sind in den folgenden Tabellen aufgelistet.

| Direkte Sequenzwiederholungen | | | |
|---|---|---|---|
| Fragment ab Base | Repeat ab Base | Größe | Repeat Sequenz 5' 3' |
| 225 | 1316 | 10 | AAGGATATTT |
| 269 | 1686 | 11 | TCTTTTTTTTT |
| 825 | 1016 | 12 | TCAAAAGTTATG |
| 1039 | 1493 | 13 | ATATGTGACGTGG |
| 1083 | 1535 | 11 | ATAGAAACAAA |
| 1085 | 1411 | 10 | AGAAACAAAA |
| 1172 | 1608 | 12 | GTGCCAACCACT |
| 1203 | 1639 | 13 | CTCTTTCCACGTG |

| Invertierte Sequenzwiederholungen | | | |
|---|---|---|---|
| Fragment ab Base | Repeat ab Base | Größe | Repeat Sequenz 5' 3' |
| 99 | 293 | 10 | CAAACATTTT |
| 275 | 275 | 10 | TTTTTAAAAA |
| 313 | 1132 | 10 | AACTTCTGTT |
| 535 | 535 | 10 | TGCATATGCA |
| 835 | 835 | 10 | TGCAGCTGCA |
| 1269 | 1370 | 11 | TGTATATCAAA |
| 1293 | 1359 | 10 | TCATCCAAAC |
| 1401 | 1401 | 10 | TTTTATAAAA |
| 1658 | 1658 | 12 | TCTGACGTCAGA |

Die Positionsangaben basieren jeweils auf der Nummerierung der Nucleotidreste gemäß SEQ ID NO:2.

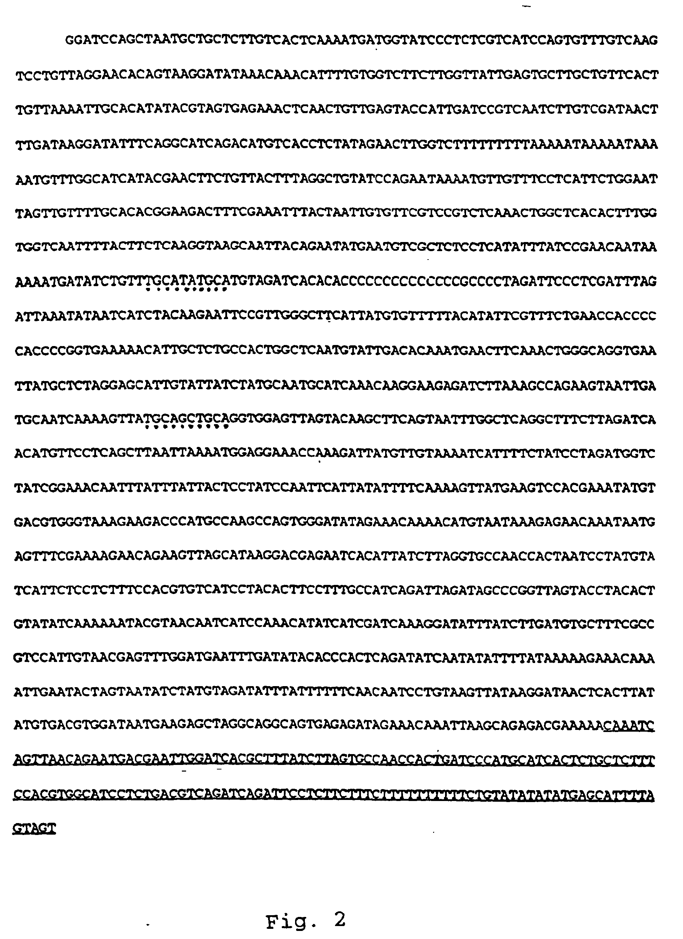

Obige Auflistung enthält insbesondere zwei 10 bp umfassende fast identische palindromische Sequenzabschnitte, die je zweimal das Motiv TGCA enthalten. Dieses Motiv liegt im Gegenstrang als ACGT vor, einer Box, die von verschiedenen Arbeitsgruppen als regulatorische Sequenz (z. B. Guliano et al., (1988), Proc. Acad. Natl. Sci. USA, 85, 7089-7093) und als Bindestelle für DNA-bindende Leucin-Zipper-Proteine (z. B. Armstrong et al., (1992), Plant Cell, 4, 525-537) identifiziert worden ist. Die Bindung von Leucin-Zipper-Proteinen ist durch die Orientierung des Motivs wahrscheinlich nicht beeinflusst. Diese Sequenzen sind durch punktierte Unterstreichung in Figur 2 markiert. Der Einfluss obiger Teilsequenzen auf die erfindungsgemäße Promotoraktivität und/oder -spezifität kann vom Fachmann z.B. anhand üblicher Deletionsexperimente überprüft werden.

Eine weitere bevorzugte Ausführungsform der Erfindung betrifft eine 5'-verkürzte Promotorsequenz (SEQ ID NO: 3). Sie umfasst eine 817 bp Promotorregion (SEQ ID NO: 5) sowie einen 292 bp 5'-untranslatierten Bereich. Der verkürzte Promotor zeigt überraschenderweise eine identische Organ- bzw. Gewebespezifität, wie der oben beschriebene längere Promotor, besitzt jedoch unterschiedliche Promotoraktivität.

Funktionell äquivalente Promotorsequenzen sind erfindungsgemäß solche Sequenzen, welche trotz abweichender Nucleotidsequenz noch die gewünschten Funktionen, i. e. Promotoraktivität und Gewebe- oder Organspezifität besitzen. Ein Maß für die Promotoraktivität ist beispielsweise die für ein bestimmtes Markergen, das unter der regulativen Kontrolle des erfindungsgemäßen Promotors steht, ermittelte Expressionsrate. Beispiele für geeignete Markergene sind das β-Glucuronidase (GUS)-Gen aus E. coli oder das Green-Fluorescence-Protein (GFP)-Gen (Baulcombe et al., (1993), Plant J., 7 (6), 1045-1053). Die Organ- bzw. Gewebespezifität lässt sich leicht durch Vergleich der an einzelnen Geweben bzw. Organen der Pflanze ermittelten Expressionsraten für obige Markergene bestimmen. Funktionelle Äquivalente umfassen im Rahmen der vorliegenden Erfindung natürlich vorkommende Varianten der hierin beschriebenen Sequenzen sowie künstliche, z.B. durch chemische Synthese erhaltene, an die Codon-Usage einer Pflanze angepaßte, künstliche Nucleotid-Sequenzen.

Unter einem funktionellen Äquivalent versteht man insbesondere auch natürliche oder künstliche Mutationen einer ursprünglich isolierten Promotorsequenz, welche weiterhin die gewünschte Funktion zeigen. Mutationen umfassen Substitutionen, Additionen, Deletionen, Vertauschungen oder Insertionen eines oder mehrerer Nukleotidreste. Somit werden beispielsweise auch solche Nucleotidsequenzen durch die vorliegende Erfindung mit umfaßt, welche man durch Modifikation der Nucleotidsequenz gemäß SEQ ID NO:1 bis 5 erhält. Ziel einer solchen Modifikation kann z.B. die weitere Eingrenzung der darin enthaltenen Promotorsequenz, oder z.B. auch die Einfügung weiterer Restriktionsenzym-Schnittstellen sein.

Funktionelle Äquivalente sind auch solche Promotorvarianten, deren Promotorfunktion, verglichen mit dem Wildtyp, abgeschwächt oder verstärkt ist.

Gegebenenfalls müssen vor der Isolierung der Promotorsequenz zunächst blattspezifisch exprimierte Gene experimentell, beispielsweise durch subtraktive Hybridisierung (bekannt aus R.A. Meyers, Molecular Biology and Biotechnology (1995), VCH, S. 698-699) identifiziert werden. Als nächstes kann eine genomische Bank aus Blättern des Donororganismus nach bekannten Verfahren erstellt werden, z.B. durch Isolierung der Gesamt-DNA, nachfolgendem Partialverdau, Verpackung von Fragmenten mit definierter Größe in Bakteriophagen, Infektion von Bakterien mit den rekombinanten Bakteriophagen und anschließende Amplifikation der genomischen Bank. Die die genomische DNA enthaltenden Phagen können dann beispielsweise auf Nylonfilter transferiert und mit einer radioaktiv markierten cDNA des zuvor identifizierten blattspezifischen Gens hybridisiert werden. Hybridisierende Phagen-DNAs können durch Autoradiographie sichtbar gemacht und danach vereinzelt werden. Zur Isolierung der Phagen-DNA können, ausgehend von je einem Einzelplaque, lytische Agarplatten angeimpft und inkubiert und die DNA in an sich bekannter Weise, z.B. durch Phenol-Chloroform-Extraktion und nachfolgende Fällung mit Ethanol, gewonnen werden. Die Fragmentlängen der Promotorbereiche der isolierten genomischen Klone können nun beispielsweise durch Southern Hybridisierungen mit einer 5'-cDNA-Probe des blattspezifisch exprimierten Genes nach unterschiedlichen Restriktionsspaltungen bestimmt werden. Ein Promotorbereich kann nun in einen geeigneten Vektor kloniert, beispielsweise in E. coli vermehrt und die komplette Nucleotid-Sequenz des Promotors durch Sequenzierung bestimmt werden. Geeignete Klonierungsvektoren sind u.a. pBR332, pUC-Serien, M13mp-Serien und pACYC184.

Zweckmäßigerweise kann der Promotor nun in einer Expressionskassette mit einem geeigneten Gen operativ verknüpft werden, so daß der Promotor die Transkription des mit ihm fusionierten Gens kontrollieren kann. Unter einer operativen Verknüpfung versteht man die sequentielle Anordnung von Promotor, codierender Sequenz, Terminator und gegebenenfalls weiteren regulativen Elementen, wobei jedes der genannten Elemente seine Funktion bei der Genexpression bestimmungsgemäß erfüllen kann.

Eine solche Expressionskassette stellt einen weiteren Gegenstand der vorliegenden Erfindung dar. Ein weiterer Gegenstand der Erfindung betrifft rekombinante Vektoren, z.B. Plasmide oder Viren, die wenigstens eine erfindungsgemäße Expressionskassette enthalten.

Grundsätzlich können die Nucleotid-Sequenzen, die der Promotorsequenz der Expressionskassette nachgeschaltet werden, alle möglichen offenen Leseraster für ein beliebiges Peptid sowie ein oder mehrere Introns enthalten. Als Beispiele seien genannt: Sequenzen für Enzyme; Sequenzen, die komplementär sind zu a) einer Genomsequenz, wobei die Genomsequenz ein offenes Leseraster sein darf; b) einem Intron; c) einer nicht kodierenden Leitsequenz; d) jeder Sequenz, die - komplementär in das Genom integriert - die Transkription, mRNA-Verarbeitungen (z.B. Splicing) oder die Translation inhibiert.

Die insertierte Nucleotid-Sequenz kann synthetisch hergestellt oder natürlich gewonnen sein oder eine Mischung aus synthetischen und natürlichen DNA-Bestandteilen enthalten. Im allgemeinen werden synthetische Nucleotid-Sequenzen mit Codons erzeugt, die von Pflanzen bevorzugt werden. Diese von Pflanzen bevorzugten Codons können aus Codons mit der höchsten Proteinhäufigkeit bestimmt werden, die in den meisten interessanten Pflanzenspezies exprimiert werden. Bei der Präparation einer Expressionskassette können verschiedene DNA-Fragmente manipuliert werden, um eine Nucleotid-Sequenz zu erhalten, die zweckmäßigerweise in der korrekten Richtung liest und die mit einem korrekten Leseraster ausgestattet ist. Für die Verbindung der DNA-Fragmente miteinander können an die Fragmente Adaptoren oder Linker angesetzt werden.

Zweckmäßigerweise sollten die erfindungsgemäßen Promotor- und die Terminator-Regionen in Transkriptionsrichtung mit einem Linker oder Polylinker, der eine oder mehrere Restriktionsstellen für die Insertion dieser Sequenz enthält, versehen werden. In der Regel hat der Linker 1 bis 10, meistens 1 bis 8, vorzugsweise 2 bis 6 Restriktionsstellen. Im allgemeinen hat der Linker innerhalb der regulatorischen Bereiche eine Größe von weniger als 100 bp, häufig weniger als 60 bp, mindestens jedoch 5 bp. Der erfindungsgemäße Promotor kann sowohl nativ bzw. homolog als auch fremdartig bzw. heterolog zur Wirtspflanze sein. Die erfindungsgemäße Expressionskassette beinhaltet in der 5'-3'-Transkriptionsrichtung den erfindungsgemäßen Promotor, eine beliebige Sequenz und eine Region für die transkriptionale Termination. Verschiedene Terminationsbereiche sind gegeneinander beliebig austauschbar.

Ferner können Manipulationen, die passende Restriktionsschnittstellen bereitstellen oder die überflüssige DNA oder Restriktionsschnittstellen entfernen, eingesetzt werden. Wo Insertionen, Deletionen oder Substitutionen wie z.B. Transitionen und Transversionen in Frage kommen, können in vitro-Mutagenese, "primerrepair", Restriktion oder Ligation verwendet werden. Bei geeigneten Manipulationen, wie z.B. Restriktion, "chewing-back" oder Auffüllen von Überhängen für "bluntends", können komplementäre Enden der Fragmente für die Ligation zur Verfügung gestellt werden.

Besonders geeignete codierende Nucleotid-Sequenzen sind Toleranz- oder Resistenz-vermittelnde Gene, Gene, die die photosynthetische Leistungsfähigkeit der Pflanze erhöhen oder Markergene, wie das β-Glucuronidase-Gen (GUS) aus Escherichia coli. Geeignete Toleranzgene sind beispielsweise solche, die die Temperatur-, Trokken- oder UV-Toleranz oder die Toleranz gegenüber Umweltschadstoffen einer Pflanze erhöhen. Geeignete Resistenzgene sind beispielsweise das bar-Gen aus Streptomyces hygroscopicus, das Resistenz gegen das Totalherbizid Phosphinothricin vermittelt, Chitinase-Gene, die Toleranz gegen Pilzinfektionen vermitteln und Riboyzym-Gene, deren RNA-Transkripte virale RNA mit hoher Spezifität erkennen und spalten können. Diese und andere Resistenzgene sind aus Transgenic Plants and Crop Improvement, in Transgenic Plants, Vol. 1, Engineering and Utilization, herausgegeben von S-d Kung und R. Wu, Academic Press, 1993, Part III, S. 243-372; sowie aus R.H. Symons (1992), Small catalytic RNAs, Ann. Rev. Biochem. 61, 641-671 bekannt. Geeignete Gene zur Erhöhung der photosynthetischen Leistungsfähigkeit sind beispielsweise die für Saccharosephosphat-Synthase (SPS) oder Fructose-1,6-bisphosphatase (FBPase) codierenden Gene.

Das fusionierte Konstrukt kann nun durch verschiedene bekannte Verfahren in pflanzliche Genome transferiert werden. Geeignete Verfahren sind beispielsweise Protoplasten-Transformation durch Polyethylenglykol-induzierte DNA-Aufnahme, Elektroporation, Sonikation oder Mikroinjektion sowie die Transformation intakter Zellen oder Gewebe durch Mikro- oder Makroinjektion in Gewebe oder Embryonen, Gewebeelektroporation, Inkubation trockener Embryonen in DNA-haltiger Lösung, biolistischer Gentransfer und besonders bevorzugt Agrobacterium-Transformation. Die genannten Verfahren sind beispielsweise in B. Jenes et al., Techniques for Gene Transfer; in Transgenic Plants, Vol. 1, Engineering and Utilization, herausgegeben von S-d Kung und R. Wu, Academic Press, 1993, S. 128-143 sowie in Potrykus (1991) Annu. Rev. Plant Physiol. Plant Molec. Biol. 42, 205-225) beschrieben. Vorzugsweise wird das fusionierte Konstrukt in einen Vektor kloniert, der geeignet ist, Agrobacterium tumefaciens zu transformieren, beispielsweise pBin19 (Bevan et al. (1980) Nucl. Acids Res. 12, 8711). Solche Vektoren und mit ihnen transformierte Mikroorganismen, insbesondere Agrobacterium sind weitere Gegenstände der vorliegenden Erfindung.

Mit einem erfindungsgemäßen Vektor transformierte Agrobakterien können dann in bekannter Weise zur Transformation von Pflanzen, insbesondere von Kulturpflanzen, wie Getreide, Mais, Soja, Reis, Baumwolle, Zuckerrübe, Canola, Sonnenblume, Flachs, Kartoffel, Tabak, Tomate, Raps, Alfalfa, Salat und den verschiedenen Baum-, Nuß- und Weispecies, verwendet werden, z.B. indem verwundete Blätter oder Blattstücke in einer Agrobakterienlösung gebadet und anschließend in geeigneten Medien kultiviert werden.

Die Verwendung erfindungsgemäßer Vektoren zur Transformation von Pflanzen ist ein weiterer Gegenstand der vorliegenden Erfindung. Die Transformation von Pflanzen durch Agrobakterien ist unter anderem bekannt aus F.F. White, Vectors for Gene Transfer in Higher Plants; in Transgenic Plants, Vol. 1, Engineering and Utilization, herausgegeben von S-d Kung und R. Wu, Academic Press, 1993, S. 15-38 und aus S.B. Gelvin, Molecular Genetics of T-DNA Transfer from Agrobacterium to Plants, gleichfalls in Transgenic Plants, S. 49-78. Aus den transformierten Zellen der verwundeten Blätter bzw. Blattstücke können in bekannter Weise transgene Pflanzen regeneriert werden, die unter Kontrolle des eingeführten Promotors das mit diesem fusionierte Gen blattspezifisch exprimieren. Solche transgenen Pflanzen, Vermehrungsgut davon sowie Pflanzenzellen, -gewebe oder -teile sind ein weiterer Gegenstand der vorliegenden Erfindung.

Die im Rahmen der vorliegenden Erfindung durchgeführten Klonierungsschritte wie z.B. Restriktionsspaltungen, Agarose-Gelelektrophorese, Reinigung von DNA-Fragmenten, Transfer von Nukleinsäuren auf Nitrozellulose und Nylonmembranen, Verknüpfen von DNA-Fragmenten, Transformation von E. coli Zellen, Anzucht von Bakterien, Vermehrung von Phagen und Sequenzanalyse rekombinanter DNA wurden wie bei Sambrook et al. (1989) Cold Spring Harbor Laboratory Press; ISBN 0-87969-309-6) beschrieben durchgeführt.

Die Erfindung wird durch die nun folgenden Beispiele erläutert, ist aber nicht auf diese beschränkt:

Die im folgenden verwendeten Bakterienstämme (E. coli, XL-1 Blue und P2 392) wurden durch die Firma Stratagene bezogen. Der zur Pflanzentransformation eingesetzte Agrobacterium tumefaciens Stamm (C58C1 mit dem Plasmid pGV 3850kan) wurde von Debleare et al. (1985, Nucl. Acid Res. 13, 4777) beschrieben. Zur Klonierung wurden die Vektoren pUC19 (Yanish-Perron (1985) Gene 33, 103-119), pBlueScript SK (Stratagene), pBin19 (Bevan (1984) Nucl. Acids Res. 12, 8711-8720) und pBI101 (Jefferson et al. (1987) EMBO J. 6, 3901-3907) verwendet.

Solanum tuberosum L Varietät Desirée wurde bezogen von Vereinigte Saatzuchten eG Ebstorf.
Nicotiana tabacum L Samsun NN wurde bezogen von Vereinigte Saatzuchten eG Ebstorf.

### Beispiel 1: Isolierung eines blattspezifischen Promotors

### 1. Isolierung eines blattspezifisch exprimierten Gens

### 1.1. Verwendete Sonde

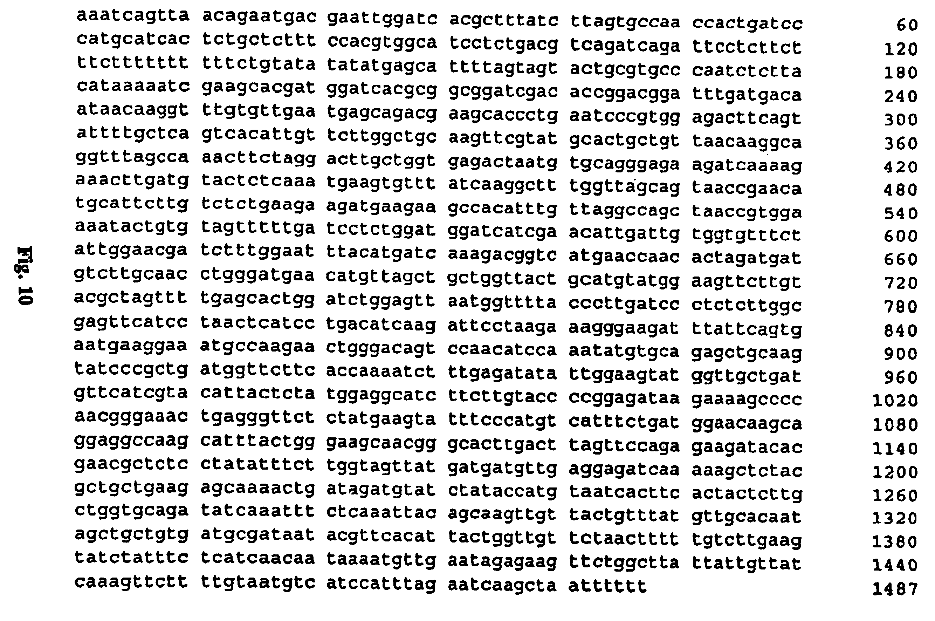

Eine mit reverser Transkriptase erzeugte cDNA-Sonde des cy-FBPase-Gens aus Kartoffel (EMBL-Nr.: X76946) wurde für die im folgenden beschriebenen Experimente verwendet. Die cDNA-Sonde (Figur 10) umfasste 1487 Nucleotide. Die für das Strukturgen (FBPase) kodierende Region umfaßt die Nucleotide 199 bis 1218.

### 1.2. Erstellung einer genomischen Bank

Zur Erstellung einer genomischen Bank aus Kartoffel (Solanum tuberosum var. Desiree) wurde Gesamt-DNA aus Kartoffelblättern nach der von Rogers et al. ((1985) Plant Mol. Biol. 5, S. 69-76) beschriebenen Methode isoliert. Anschließend wurden 300 µg der DNA mit dem Restriktionsenzym Sau3A partialverdaut und die Fragmente zwischen 12 und 20 kb mittels Saccharosegradientenzentrifugation isoliert, dialysiert und durch Ausschütteln mit Butanol konzentriert.

Die DNA wurde in von Stratagene (11099 North Torrey Pines Road, La Jolla, CA 92037, USA) bezogene BamHI verdaute EMBL3-Arme nach Herstellerangaben ligiert und anschließend in vitro verpackt (Gigapack II Gold Verpackungsextrakte, Stratagene, nach Herstellerangaben). E. coli Bakterien des Stammes P2 392 (Stratagene) wurden mit den rekombinanten Lambdaphagen infiziert, der Titer der Bank bestimmt und anschließend die Bank amplifiziert.

### 1.3.Screenen der genomischen Bank und Isolierung des cy-FBPase Gens

Zur Isolierung eines das cy-FBPase Gen umfassenden genomischen Klons wurden 3 x 10⁵ Phagen plattiert. Nach Transfer der Phagen auf Nylonfilter (Hybond N, Amersham Buchler) wurden die Filter zur Fixierung 2 Stunden bei 80°C gebacken. Anschliessend wurden sie bei 42°C in Hypo-Hybond-Puffer prähybrisiert.

### 1 1 Hypo-Hybond-Puffer enthält:

| | |
|---|---|
| 250 ml | 1M Natriumphosphatpuffer pH 7,2 |
| 50 ml | 5M NaCl |
| 2 ml | 0,5M EDTA pH 8,0 |
| 2 ml | Heringssperma-DNA sonifiziert 1 mg/l |
| 400 ml | Formamid |
| 50 g | PEG 6000 |
| 70 g | SDS |
| 200 ml | Wasser |

Die mit High-Prime (Boehringer Mannheim) radioaktiv markierte Probe der cy-FBPase aus Kartoffel wurde nach 5 minütiger Denaturierung bei 95°C in die Prähybridisierungslösung gegeben. Die Filter wurden über Nacht bei 42°C hybridisiert. Nach Abziehen der radioaktiven Hybridisierungslösung wurden die Filter 20 min bei 42°C in 2X SSC (einem NaCl/NaCitrat-Puffer), 0,1% SDS gewaschen. Anschliessend wurde erneut 20 min bei gleicher Temperatur mit 1X SSC, 0,1% SDS gewaschen. Danach wurde ein Film auf die Filter aufgelegt und über Nacht bei -70°C exponiert.

Es wurden 4 hybridisierende Phagen-DNAs durch Autoradiographie sichtbar gemacht und vereinzelt. Ausgehend von je einem Einzelplaque wurde je eine lytische Agarplatte angeimpft, über Nacht bei 37°C inkubiert und die Phagen am nächsten Tag mit 10 ml Phagenpuffer (SM) abgeschwemmt. Anschließend wurde der Phagenüberstand mit Chloroform versetzt und die Bakterien wurden abzentrifugiert. Zum Überstand wurden je eine Spatelspitze DNase und RNase gegeben und 30 min bei 37°C inkubiert. Nach Zugabe von 100 µl 0,5 M EDTA und 200 µl 10%-iger SDS-Lösung wurde der Ansatz für weitere 20 Minuten bei 65°C inkubiert. Dann wurden 4,5 ml 3M Kaliumacetatlösung pH 4,8 zugegeben, der Ansatz gemischt und abzentrifugiert. Der Überstand wurde anschließend mit Phenol:Chloroform:Isoamylalkohol (Volumenverhältnis 25:24:1) ausgeschüttelt. Nach Extraktion wurde die DNA durch Zugabe von zwei Volumina Ethanol aus dem Überstand gefällt und das erhaltene Sediment in 600 µl TE-RNase gelöst.

### 2. Lokalisierung des Promotors

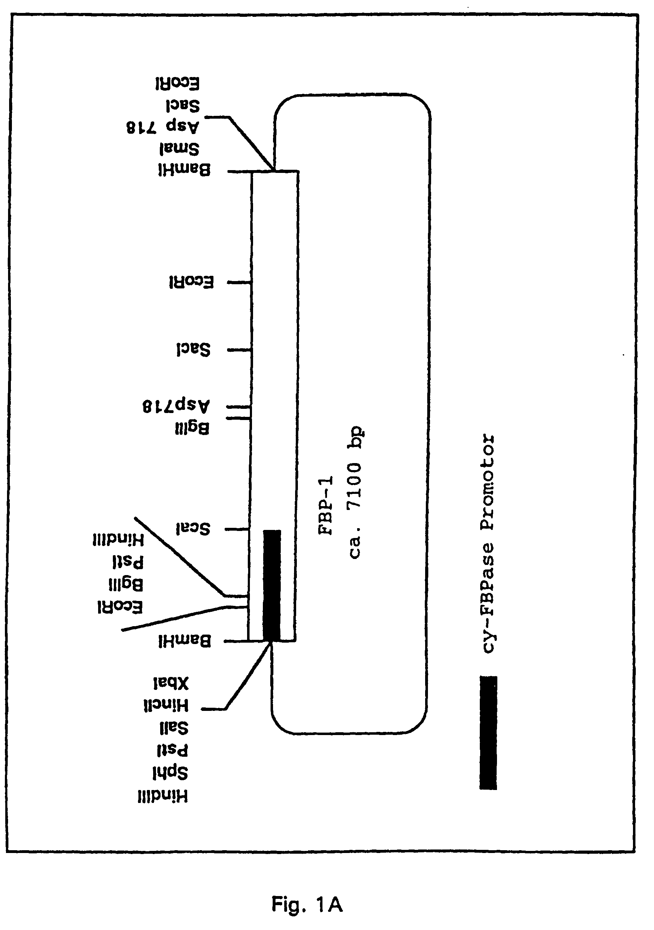

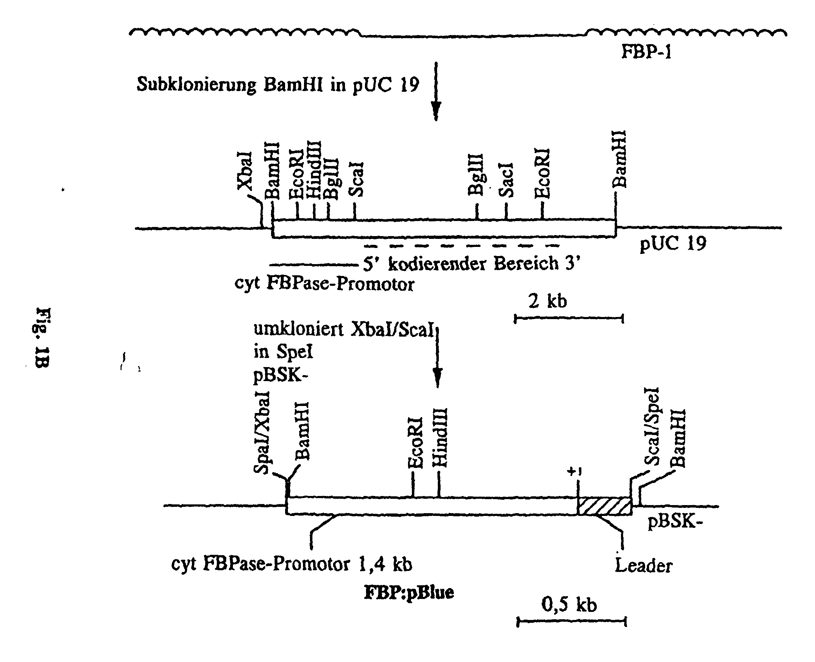

Die Fragmentlängen der Promotorbereiche der 4 isolierten Klone wurden durch Southernhybridisierungen mit einer 5'-cDNA-Probe nach unterschiedlichen Restriktionsspaltungen bestimmt. Klon FBP-1 wurde für weitere Analysen ausgewählt. Ein ca. 7100 b BamHI-Fragment des Klons FBP-1 wurde zur weiteren Charakterisierung in die BamHI-Schnittstelle des Vektors pUC19 kloniert. Durch Sequenzierung, Southernhybridisierung und Restriktionsanalyse konnte der Promotorbereich auf ein 1724 Basenpaar-Fragment eingeschränkt werden (Figur 1A). Als Sonde für die Southernhybridisierung dienten die 5'-342 bp (*Hinc*II/*Eco*RI) und 3'-216 bp (*EcoR*I/*EcoR*V) Subfragmente der cDNA der cy-FBPase. Aus der Sequenz der cDNA der cytosolischen FBPase war bekannt, daß das Restriktionsenzym ScaI im nicht kodierenden 5'-Bereich der cDNA schneidet. Im genomischen Klon FBP-1 war eine singuläre ScaI-Schnittstelle zu finden. Durch die Sequenzinformation über den genomischen Klon konnte gezeigt werden, daß es sich hierbei um die der cDNA entsprechende Schnittstelle handelte. Sie wurde benutzt, um den Promotorbereich von der kodierenden Region abzutrennen. Dazu wurde die Promotorregion XbaI/ScaI aus dem Klon FBP-1 ausgeschnitten, die Enden aufgefüllt und in den Vektor pBlueScript-SK (pBSK-) ligiert, der zuvor SpeI geschnitten und aufgefüllt wurden war (Bezeichnung FBP:pBlue). Die Herstellung von FBP:pBlue ist in Figur 1B schematisch dargestellt. Anschliessend wurde die komplette DNA-Sequenz durch Sequenzierung bestimmt (Figur 2).

### Beispiel 2: Herstellung eines Transformationsvektors

### 1. Herstellung des Plasmids FBP:GUS

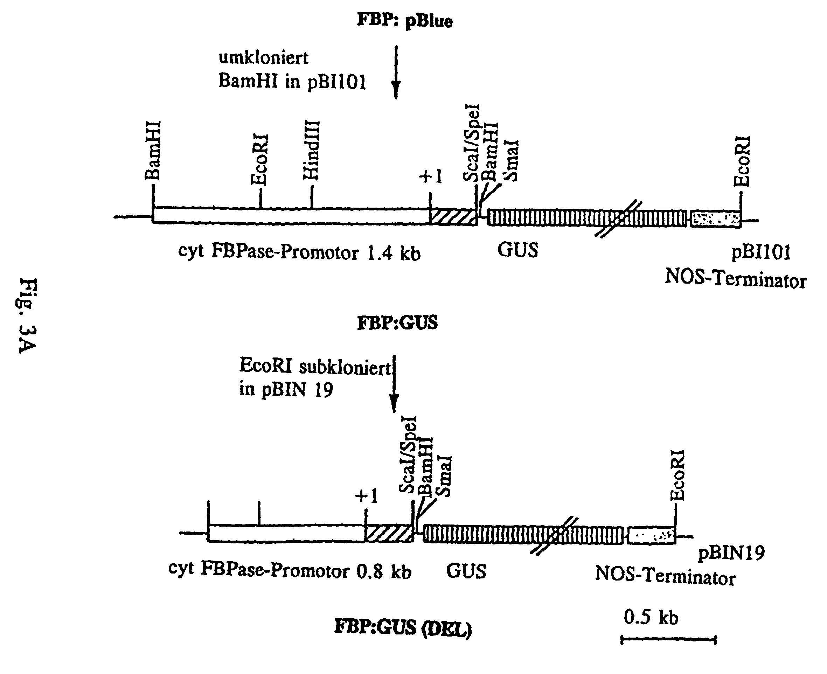

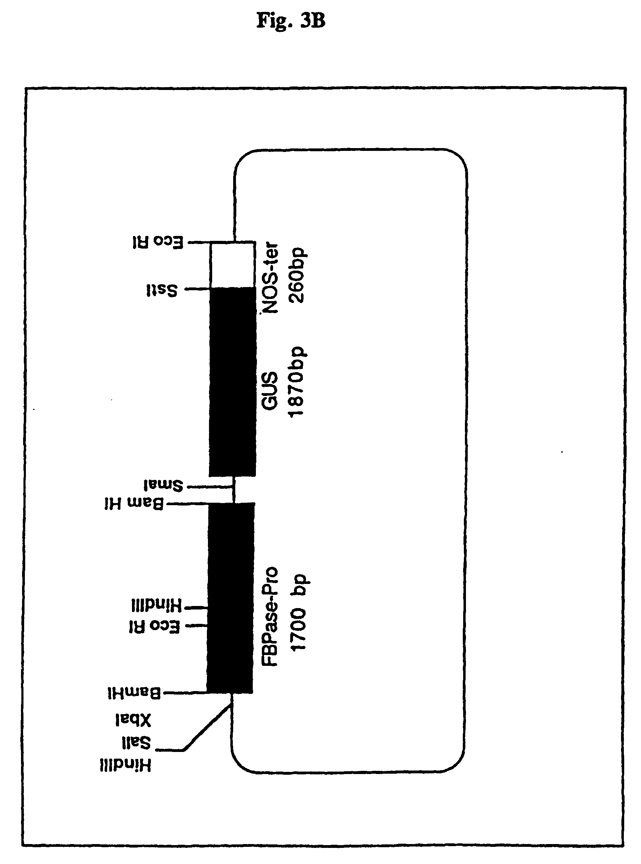

Die Expressionseigenschaften des neuen Promotors wurden durch Markergenexperimente analysiert. Zu diesem Zweck wurde der cy-FBPase-Promotor mit dem β-Glucuronidase-Gen (GUS) aus E. coli fusioniert. Der Promotor wurde als BamHI-Fragment aus dem Plasmid FBP:pBlue isoliert und in die BamHI-Schnittstelle des Expressionsvektors pBI101 kloniert (Figur 3A) (Jefferson et al., (1987), EMBO J. 6, 3901-3907). Das resultierende Plasmid FBP:GUS (Figur 3B) wurde anschließend zur Transformation von Agrobacterium tumefaciens eingesetzt.

### 2. Herstellung des Deletionskonstruktes FBP:GUS(DEL)

Es wurde ein Deletionskonstrukt hergestellt, das etwa 1,1 kb der Promotorsequenz umfasst. Dazu wurde aus FBP:GUS mittels EcoRI-Verdau ein Fragment ausgeschnitten, das das GUS-Gen, den NOS-Terminator sowie 1100 bp des Promotors umfasst. Dieses Fragment wurde isoliert, gereinigt und in die EcoRI-Schnittstelle des Vektors pBin 19 ligiert (Figur 3A). Die Orientierung des Fragments im pBin 19 wurde durch Spaltung mit BamHI überprüft. Mit diesem Vektor wurde Agrobacterium tumefaciens ebenfalls transformiert.

### Beispiel 3: Transformation von Agrobacterium tumefaciens

Die Transformation von Agrobacterium tumefaciens wurde entsprechend der Methode von Höfgen und Willmitzer (Nucl. Acid Res. (1988) 16, 9877) ausgeführt. Die Anzucht der Agrobakterien erfolgte in YEB-Medium (Vervliet et al., J. Gen. Virol. (1975) 26, 33).

### Beispiel 4: Transformation des cy-FBPase-Promotors in Tabak- und Kartoffelpflanzen und Analyse der Expression

### 1.1. Tabaktransformation

Zur Transformation von Tabakpflanzen (Nicotiana tabacum L. cv. Samsun NN) wurden 10 ml einer unter selektion gewachsenen Übernachtkultur von mit FBP:GUS bzw. FBP:GUS(DEL) transformiertem Agrobacterium tumefaciens abzentrifugiert, der Überstand verworfen, und die Bakterien im gleichen Volumen Antibiotika-freien Mediums resuspendiert. In einer sterilen Petrischale wurden Blattscheiben steriler Pflanzen (Durchmesser ca. 1 cm) in dieser Bakterienlösung gebadet. Anschließend wurden die Blattscheiben in Petrischalen auf MS-Medium (Murashige und Skoog, Physiol. Plant. (1962) 15,473) mit 2% Saccharose und 0,8% Bacto-Agar ausgelegt. Nach 2-tägiger Inkubation im Dunkeln bei 25°C wurden sie auf MS-Medium mit 100 mg/l Kanamycin, 500 mg/l Clarofan, 1 mg/l Benzylaminopurin (BAP), 0,2 mg/l Naphthylessigsäure (NAA), 1,6% Glukose und 0,8% Bacto-Agar übertragen und die Kultivierung (16 Stunden Licht/ 8 Stunden Dunkelheit) fortgesetzt. Wachsende Sprosse wurden auf hormonfreies MS-Medium mit 2% Saccharose, 250 mg/l Clarofan und 0,8% Bacto-Agar überführt.

### 1.2. Kartoffeltransformation

20 kleine, mit dem Skalpell verwundete Blätter einer Kartoffel-Sterilkultur wurden in 10 ml MS-Medium mit 2% Saccharose gelegt, welches 50 µl einer mit FBP:GUS bzw. FBP:GUS(DEL) transformierten, unter Selektion gewachsenen Agrobacterium tumefaciens Übernachtkultur enthielt. Nach 5 minütigem, leichtem Schütteln wurden die Petrischalen bei 25°C im Dunkeln inkubiert. Nach zwei Tagen wurden die Blätter auf MS-Medium mit 1,6% Glukose, 2 mg/l Zeatinribose, 0,02 mg/l Giberellinsäure, 500 mg/l Clarofan, 50 mg/l Kanamycin und 0,8% Bacto-Agar ausgelegt. Nach einwöchiger Inkubation bei 25°C und 3000 LUX wurde die Clarofankonzentration im Medium halbiert. Eine weitere Kultivierung erfolgte nach bekannten Methoden (Rocha-Sosa et al. (1989) EMBO J. 8, 23-29).

### 1.3. Expressionsanalyse des cy-FBPase-Promotors in transgenen Tabak- und Kartoffelpflanzen

Von den transformierten Tabak- und Kartoffelpflanzen wurden jeweils 60 transformierte Pflanzen regeneriert und die β-Glucuronidase-Aktivität bestimmt. Der β-Glucuronidasenachweis erfolgte wie von Martin et al. (1992) in: The GUS Reporter System as a Tool to Study Plant Gene Expression in: GUS Protocols: Using the GUS Gene as a Reporter of Gene Expression, Academic Press, S. 23-43 beschrieben. Zur genaueren Analyse der Expression wurden 40 Tabakpflanzen und 21 Kartoffelpflanzen ausgewählt. Nach Transfer der transformierten Pflanzen in das Gewächshaus wurde die organspezifische Expression der β-Glucuronidase bestimmt.

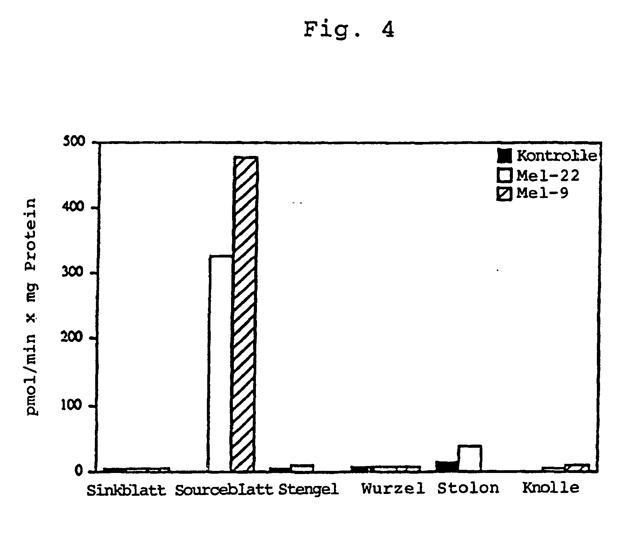

In Figur 4 ist ein Vergleich der Enzymaktivitäten in unterschiedlichen Kartoffelgeweben dargestellt. Aus den Daten wird ersichtlich, daß der Promotor eine blattspezifische Expression des Reportergens vermittelt.

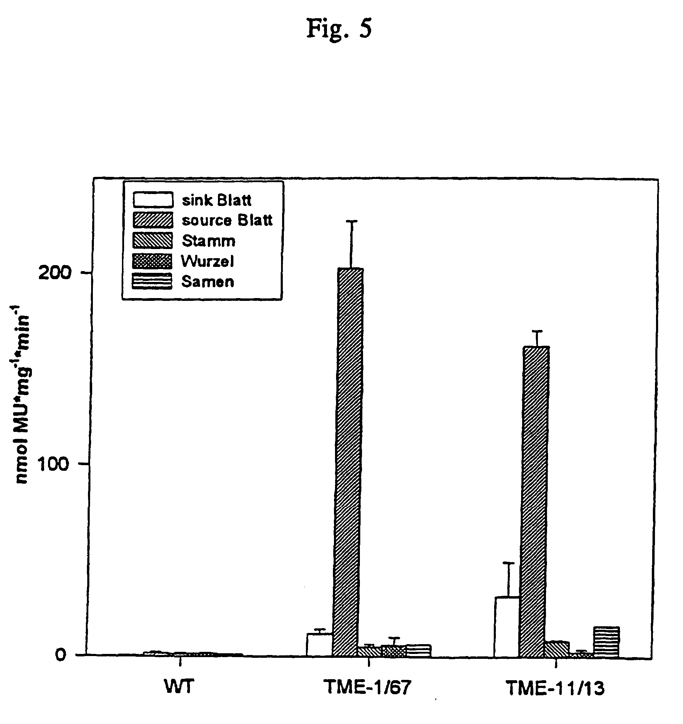

In Figur 5 wird die GUS-Aktivität in verschiedenen Organen vom Wildtyp und transgenen Tabakpflanzen verglichen, die das GUS-Gen unter der Kontrolle verschiedener erfindungsgemäßer Promotoren tragen. TME-1/67 bezeichnet eine mit FBP:GUS transformierte Pflanze. TME-11/13 bezeichnet eine mit FBP:GUS(DEL) transformierte Pflanze.

Die Bestimmung der GUS-Aktivität in den Organen Sink- und Source-Blatt, Stamm und Wurzel von 9 Wochen alten Tabakpflanzen sowie Samen ergab, dass in beiden Transformationslinien die höchste Aktivität in Source-Blättern zu finden ist, in Sink-Blättern eine deutlich geringere. Die Messwerte in Sink-Blättern waren innerhalb eines Blattes stets sehr unterschiedlich. Die Aktivität in Stamm und Wurzel lag nur unerheblich über der Hintergrundaktivität, die in Wildtyptabak gemessen wurde. Der Promotor ist in diesen Geweben nicht aktiv. In Tabaksamen konnte im Vergleich zu Samen des Wildtyps leicht erhöhte Aktivitäten gemessen werden, obwohl im "Northern-Blot" keine mRNA nachzuweisen war. Die GUS-Aktivität in Samen wurde auch bei Inkubation von Samenhomogenat in X-Gluc-Lösung gefunden. Wildtyp-Samen zeigten hier keine Färbung (nicht gezeigt). Möglicherweise ist der Promotor im Verlauf der Samenentwicklung aktiv und nicht in den reifen Samen selber. Die GUS-Aktivität könnte auf gespeichertes Protein zurückzuführen sein. Je nach Pflanze war in den Blättern eine um einen Faktor von etwa 10 bis 50 erhöhte Aktivität im Vergleich zum Samen feststellbar.

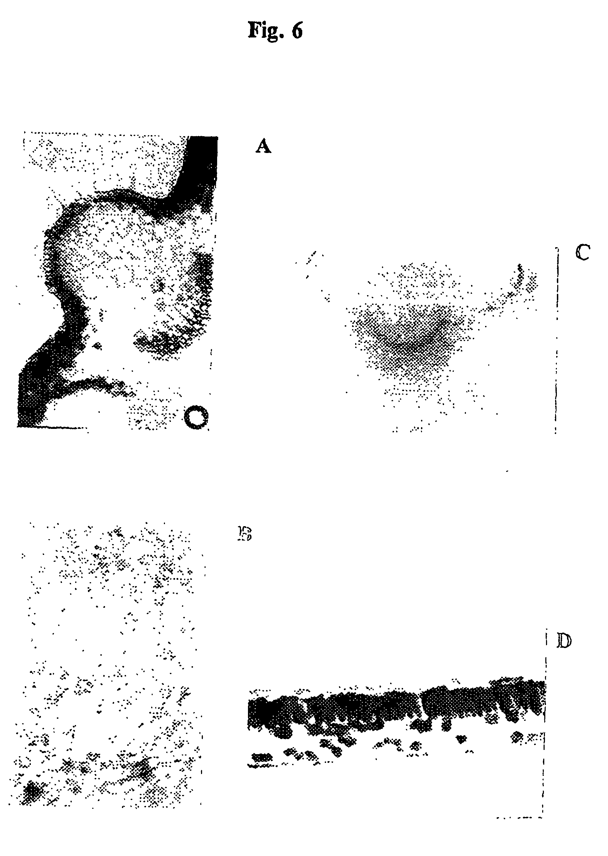

In Figur 6 wird die Zellspezifität des erfindungsgemäßen Konstruktes FBP:GUS veranschaulicht. Identische histologische Befunde erhält man mit dem verkürzten Promotorkonstrukt FBP:GUS(DEL). Um die Zellspezifität des cyt FBPase-Promotors im Blatt genauer zu untersuchen, wurden Blatt-Querschnitte von voll entfalteten Tabakblättern im Gewächshaus gezogener 8 Wochen alter Pflanzen angefertigt. Die Schnitte wurden in 3%igem Paraformaldehyd für 20 min fixiert, über Nacht in X-Gluc(5-Brom-4-chlor-3-indolyl-β-D-glucuronid)-Lösung gefärbt und anschließend das Chlorophyll mit 7%-igem Ethanol entfernt. Zusätzlich wurde die Epidermis vom Mesophyll abgezogen und getrennt inkubiert, um Kontaminationen durch Dibrom-dichlor-indigo, das von beschädigten Mesophyllzellen in die Färbelösung abgegeben worden war, zu vermeiden.

In Figur 6 (A) ist ein Querschnitt durch das zentrale Leitgewebe eines Source-Blattes gezeigt. Es ist deutlich zu erkennen, dass im zentralen Leitgewebe nur die Mesophyllzellen an der Oberseite gefärbt waren. Das parenchymatische Gewebe, sowie Xylem, Phloem und andere hier lokalisierte Gewebe waren nicht gefärbt. Einige der Epidermiszellen schienen gefärbt zu sein. Hierbei handelte es sich wahrscheinlich nicht um Aktivität in den Zellen, denn die isoliert inkubierte Epidermis zeigte keine GUS-Aktivität in Trichomen, Stomata und Epidermiszellen (B). Die Färbung der Epidermiszellen in (A) könnte an Kontaminationen aus ausgeschnittenen Mesophyllzellen liegen oder daran, dass die Schnitte mehrschichtig waren und hinter den Epidermiszellen liegende Mesophyllzellen durchschienen. In der Petiole fand sich keine Blaufärbung (C). Im Querschnitt durch das Mesophyll zeigte sich sehr starke Expression im Palisadenparenchym und etwas geringere im Schwammparenchym (D).

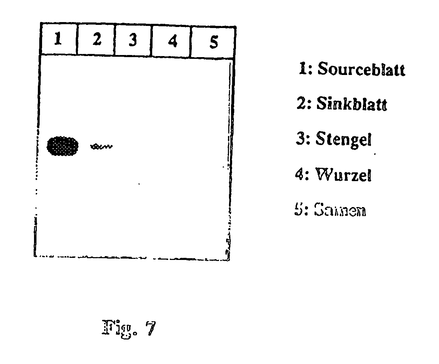

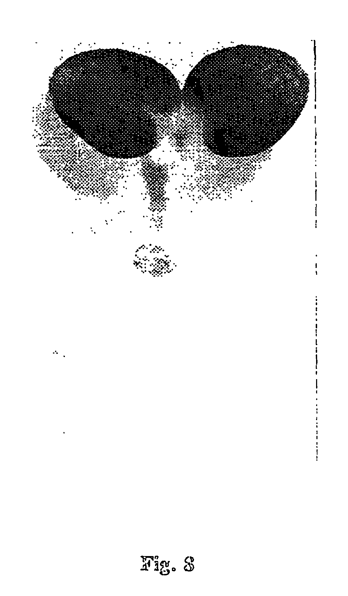

Zur genaueren Untersuchung der Expression wurde die Gesamt-RNA aus unterschiedlichen Organen von Tabakpflanzen (transformiert mit FBP:GUS) isoliert und GUS-spezifische Transkripte mittels Northern-Analysen nachgewiesen (Figur 7). Die Isolierung erfolgte wie bei Logemann et al. (Anal. Biochem. (1987) 163,21) beschrieben. Für die Analyse wurden jeweils 20 bis 40 µg RNA in einem Formaldehyd-haltigen 1,5%igen Agarosegel aufgetrennt. Nach elektrophoretischer Auftrennung der RNA Moleküle wurde die RNA mittels Kapillartransfer auf eine Nylonmembran übertragen. Der Nachweis spezifischer Transkripte wurde wie bei Amasino (Anal. Biochem. (1986) 152, 304) beschrieben durchgeführt. Die als Sonde eingesetzten cDNA-Fragmente wurden mit einem Random Primed DNA Labeling Kit (Boehringer, Mannheim) radioaktiv markiert. GUS-spezifische Transkripte konnten nur in Sink- und Source Blättern nachgewiesen werden, wohingegen kein positives Signal in Stengeln, Wurzeln und Samen identifiziert werden konnte. Der Vergleich der in Sink- und Source-Blättern erhaltenen β-Glucuronidase Aktivität zeigt, daß eine vielfach höhere Aktivität in Source-Blättern vorliegt. Histochemische Untersuchungen von Keimlingen ergaben, daß auch in frühen Entwicklungsstadien von Tabakpflanzen eine gleichmäßige blattspezifische Expression gewährleistet ist (Figur 8).

### Beispiel 5: Erstellung des Vektors pBin-FBP

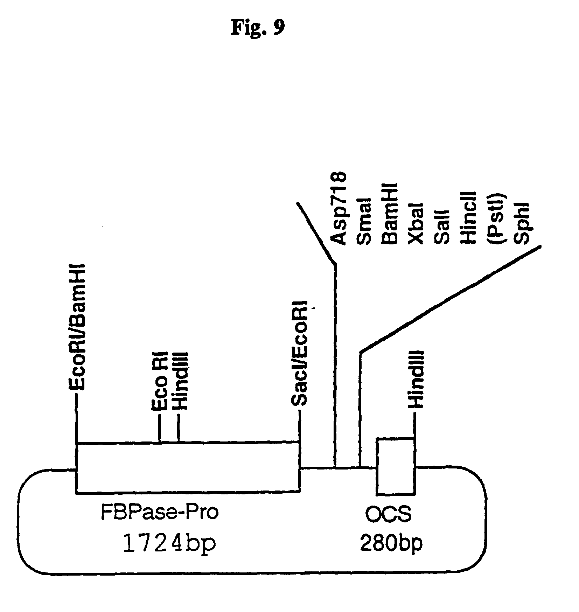

Das Promotorfragment wurde mit BamHI/SacI aus pUC 19 ausgeschnitten; die Enden wurden mit T4-DNA-Polymerase aufgefüllt. Anschließend wurde das Fragment über ein 1%-iges TAE-Agarosegel elektrophoretisch vom Vektor pUC19 getrennt und mit Glassmilch (BIO 101.1070 Joshua Way, Vista, CA 92083, USA) gereinigt. Es wurde dann in ein EcoRI geschnittenes und mit Klenow-Enzym behandeltes pBin19-Derivat ligiert. Dieses Derivat enthielt somit eine den cy-FBPase-Promotor und die Terminatorsequenz der Octopin-Synthase aus Agrobakterium tumefaciens umfassende Expressionskassette (Figur 9).

## Patentansprüche

1. Promotor, **dadurch gekennzeichnet, dass** er in Pflanzen eine mesophyllspezifische Expression einer von ihm kontrollierten codierenden Nucleotidsequenz bewirkt, und im wesentlichen eine Promotorsequenz einer cytosolischen Fructose-1,6-Bisphosphatase von Pflanzen der Gattung Solanum umfasst, wobei der Promotor durch folgenden Satz Sequenzwiederholungen **gekennzeichnet** ist:
5'-TCAAAAGTTATG-3' und
5'-AGAAACAAAA-3'

2. Promotor nach Anspruch 1, **dadurch gekennzeichnet, dass** er die Sequenz des Promotors cytosolischer Fructose-1,6-Bisphosphatase aus blattspezifischen Mesophyllzellen von Pflanzen der Gattung Solanum umfasst.

3. Promotor nach einem der vorherigen Ansprüche, **dadurch gekennzeichnet, dass** er die Nukleotidsequenz SEQ ID NO:1, SEQ ID NO:2 oder SEQ ID NO:3 umfasst.

4. Promotor nach Anspruch 3, umfassend eine Nucleotidsequenz von Nucleotid +1 bis +1428 gemäß SEQ ID NO:4 oder +1 bis +817 gemäß SEQ ID NO:5.

5. Expressionskassette, **dadurch gekennzeichnet, dass** sie wenigstens eine in einem der Ansprüche 1 bis 4 definierte Promotorsequenz umfasst.

6. Expressionskassette nach Anspruch 5, **dadurch gekennzeichnet, dass** sie eine codierende Nucleotid-Sequenz umfasst, die in Pflanzen Resistenz oder eine Steigerung der Photosyntheseleistung der Pflanze vermittelt.

7. Rekombinanter Vektor, **dadurch gekennzeichnet, dass** er eine in Anspruch 5 oder 6 definierte Expressionskassette umfasst.

8. Mikroorganismus, **dadurch gekennzeichnet, dass** er einen rekombinanten Vektor nach Anspruch 7 enthält.

9. Mikroorganismus nach Anspruch 8 aus der Gattung *Agrobacterium* und insbesondere der Art *Agrobacterium tumefaciens.*

10. Verwendung eines Vektors nach Anspruch 7 oder eines Mikroorganismus nach Anspruch 8 oder 9 zur Transformation von Pflanzen, Pflanzenzellen, -geweben oder -teilen.

11. Verwendung nach Anspruch 10, **dadurch gekennzeichnet, dass** die transformierte Pflanze ausgewählt ist unter Kulturpflanzen, wie Getreide, Mais, Soja, Reis, Baumwolle, Zuckerrübe, Canola, Sonnenblume, Flachs, Kartoffel, Tabak, Tomate, Raps, Alfalfa, Salat und den verschiedenen Baum-, Nuß- und Weinspecies,

12. Verwendung eines Promotors nach einem der Ansprüche 1 bis 4,
a) zur Herstellung eines pflanzlichen Bioreaktors;
b) zur Veränderung der Zusammensetzung von Pflanzeninhaltsstoffen;
c) zur Manipulation des pflanzlichen Stoffwechsels; und
d) zur Übertragung von Resistenzgenen auf Pflanzen.

13. Transgene Pflanze, transformiert mit einem Vektor gemäß Anspruch 7 oder mit einem Mikroorganismus gemäß einem der Ansprüche 8 oder 9, oder transgene Zellen, Gewebe, Teile oder transgenes Vermehrungsgut davon.

14. Transgene Pflanze nach Anspruch 13, ausgewählt unter Kulturpflanzen, wie Getreide, Mais, Soja, Reis, Baumwolle, Zuckerrübe, Canola, Sonnenblume, Flachs, Kartoffel, Tabak, Tomate, Raps, Alfalfa, Salat und den verschiedenen Baum-, Nuß- und Weinspecies.

15. Verfahren zur Herstellung von transgenen Pflanzen nach einem der Ansprüche 13 oder 14, **dadurch gekennzeichnet, dass** man Pflanzenzellen, -gewebe oder-teile oder Protoplasten mit einem Vektor gemäß Anspruch 7 oder mit einem Mikroorganismus gemäß einem der Ansprüche 8 oder 9 transformiert, die transformierten Zellen, Gewebe, Pflanzenteile oder Protoplasten in einem Wachstumsmedium kultiviert und gegebenenfalls aus der Kultur Pflanzen regeneriert.

16. Verfahren zur Isolierung, eines mesophyllspezifischen Promotors nach Anspruch 1, **dadurch gekennzeichnet, dass** man
a) eine pflanzliche genomische Bank mit cDNA von cytFBPase, EMBL Nr, X76946, hybridisiert,
b) positive Klone isoliert und
c) die isolierten Klone auf Promotor-Aktivität testet.

17. Nucleinsäuresequenz, ausgewählt unter SEQ ID NO:1, 2, 3, 4 und 5.

18. Funktionales Äquivalent eines Promotors nach einem der Ansprüche 1 bis 4, welches in Pflanzen eine mesophyllspezifische Expression einer davon kontrollierten codierenden Nucleotidsequenz bewirkt, wobei das funktionale Äquivalent ausgewählt ist unter
a) natürlich vorkommenden Varianten der Sequenzen gemäß SEQ ID NO: 1 bis 5;
b) von Sequenzen gemäß SEQ ID NO: 1 bis 5 abgeleiteten, an die Codon-Usage einer Pflanze angepaßten künstlichen Sequenzen;
c) von Sequenzen gemäß SEQ ID NO: 1 bis 5 durch Substitution, Addition, Deletion, Vertauschung oder Insertion eines oder mehrerer Nucleotidreste gemäß SEQ ID NO: 1 bis 5 erhaltenen Mutanten, welche wenigstens eine der folgenden, mindestens 10 bp langen
Repeat-Sequenzen aufweisen:
| Repeat-Sequenz 5' 3' |
|---|
| AAGGATATTT |
| TCTTTTTTTTT |
| ATATGTGACGTGG |
| ATAGAAACAAA |
| GTGCCAACCACT |
| CTCTTTCCACGTG |
| Repeat-Sequenz 5' 3' |
|---|
| CAAACATTTT |
| TTTTTAAAAA |
| AACTTCTGTT |
| TGCATATGCA |
| TGCAGCTGCA |
| TGTATATCAAA |
| TCATCCAAAC |
| TTTTATAAAA |
| TCTGACGTCAGA |

## Claims

1. A promoter which causes a mesophyll-specific expression, in plants, of an encoding nucleotide sequence under the control of said promoter, and essentially comprises a promoter sequence of a cytosolic fructose-1,6-bisphosphatase from plants of the genus Solanum, the promoter being **characterized by** the following set of sequence repeats:
5'-TCAAAAGTTATG-3' and
5'-AGAAACAAAA-3'.

2. The promoter according to claim 1, comprising the sequence of the promoter of cytosolic fructose-1,6-bisphosphatase from leaf-specific mesophyll cells of plants of the genus Solanum.

3. The promoter according to either of the preceding claims, comprising the nucleotide sequence SEQ ID NO:1; SEQ ID NO:2 or SEQ ID NO:3.

4. The promoter according to claim 3 comprising a nucleotide sequence from nucleotide +1 to +1428 in accordance with SEQ ID NO:4 or +1 to +817 in accordance with SEQ ID NO:5.

5. An expression cassette comprising at least one promoter sequence defined in any of claims 1 to 4.

6. The expression cassette according to claim 5, comprising an encoding nucleotide sequence which imparts resistance to plants or an increase in the photosynthesis rate of the plant.

7. A recombinant vector comprising an expression cassette defined in claim 5 or 6.

8. A microorganism comprising a recombinant vector according to claim 7.

9. The microorganism according to claim 8 from the genus *Agrobacterium* and, in particular, of the species *Agrobacterium tumefaciens*.

10. The use of a vector according to claim 7 or of a microorganism according to claim 8 or 9 for the transformation of plants, plant cells, plant tissues or parts of plants.

11. The use according to claim 10, wherein the transformed plant is selected from amongst crop plants such as cereals, maize, soya, rice, cotton, sugarbeet, canola, sunflower, flax, potato, tobacco, tomato, oilseed rape, alfalfa, lettuce and the various tree, nut and grapevine species.

12. The use of a promoter according to any of claims 1 to 4
a) for the generation of a plant bioreactor;
b) for altering the profile of plant constituents;
c) for manipulating the plant metabolism; and
d) for the transfer of resistance genes to plants.

13. A transgenic plant, transformed with a vector according to claim 7 or with a microorganism according to either of claims 8 and 9, or transgenic cells, tissue, parts or transgenic propagation material thereof.

14. The transgenic plant according to claim 13, selected from amongst crop plants such as cereals, maize, soya, rice, cotton, sugarbeet, canola, sunflower, flax, potato, tobacco, tomato, oilseed rape, alfalfa, lettuce and the various tree, nut and grapevine species.

15. A method of producing transgenic plants according to either of claims 13 and 14, which comprises transforming plant cells, plant tissue, parts of plants or protoplasts with a vector according to claim 7 or with a microorganism according to either of claims 8 and 9, culturing the transformed cells, tissue, parts of plants or protoplasts in a growth medium and, if appropriate, regenerating plants from the culture.

16. A method of isolating a mesophyll-specific promoter according to claim 1 which comprises
a) hybridizing a plant genome library with cDNA from cytFBPase, EMBL No. X76946,
b) isolating positive clones and
c) testing the isolated clones for promoter activity.

17. A nucleic acid sequence selected from amongst SEQ ID NO:1, 2, 3, 4 and 5.

18. A functional equivalent of a promoter according to any of claims 1 to 4 which causes a mesophyll-specific expression, in plants, of an encoding nucleotide sequence under the control of said promoter, the functional equivalent being selected from
a) naturally occurring variants of the sequences according to SEQ ID NO: 1 to 5;
b) artificial sequences derived from sequences according to SEQ ID NO: 1 to 5 and adapted to the codon usage of a plant;
c) mutants obtained from sequences according to SEQ ID NO: 1 to 5 by substitution, addition, deletion, exchange or insertion of one or more nucleotide residues according to SEQ ID NO: 1 to 5 and comprising at least one of the following repeat sequences which are at least 10 bp in length:
| repeat sequence 5' 3' |
|---|
| AAGGATATTT |
| TCTTTTTTTTT |
| ATATGTGACGTGG |
| ATAGAAACAAA |
| GTGCCAACCACT |
| CTCTTTCCACGTG |
| repeat sequence 5' 3' |
|---|
| CAAACATTTT |
| TTTTTAAAAA |
| AACTTCTGTT |
| TGCATATGCA |
| TGCAGCTGCA |
| TGTATATCAAA |
| TCATCCAAAC |
| TTTTATAAAA |
| TCTGACGTCAGA |

## Revendications

1. Promoteur, **caractérisé en ce qu'**il provoque chez des plantes une expression, spécifique du mésophylle, d'une séquence de nucléotides codante, sous le contrôle d'un tel promoteur, et comprend essentiellement une séquence de promoteur d'une fructose-1,6-bisphosphatase cytosolique de plantes appartenant au genre *Solanum,* le promoteur étant **caractérisé par** la série suivante de répétitions de séquences :
5'-TCAAAAGTTATG-3' et
5'-AGAAACAAAA-3'.

2. Promoteur selon la revendication 1, **caractérisé en ce qu'**il comprend la séquence du promoteur de fructose-1,6-bisphosphatase cytosolique de cellules de mésophylle spécifique de la feuille de plantes appartenant au genre *Solanum.*

3. Promoteur selon l'une quelconque des revendications précédentes, **caractérisé en ce qu'**il comprend la séquence de nucléotides SEQ ID n° 1, SEQ ID n° 2 ou SEQ ID n° 3.

4. Promoteur selon la revendication 3, comprenant une séquence de nucléotides allant du nucléotide +1 au nucléotide +1428 selon SEQ ID n° 4 ou du nucléotide +1 au nucléotide +817 selon SEQ ID n° 5.

5. Cassette d'expression, **caractérisée en ce qu'**elle comprend au moins une séquence de promoteur définie dans l'une quelconque des revendications 1 à 4.

6. Cassette d'expression selon la revendication 5, **caractérisée en ce qu'**elle comprend une séquence de nucléotides codante qui induit chez des plantes une résistance ou une augmentation du pouvoir de photosynthèse de la plante.

7. Vecteur recombinant, **caractérisé en ce qu'**il comprend une cassette d'expression définie dans la revendication 5 ou 6.

8. Micro-organisme, **caractérisé en ce qu'**il contient un vecteur recombinant selon la revendication 7.

9. Micro-organisme selon la revendication 8, appartenant au genre *Agrobacterium* et en particulier à l'espèce *Agrobacterium tumefaciens*.

10. Utilisation d'un vecteur selon la revendication 7 ou d'un micro-organisme selon la revendication 8 ou 9, pour la transformation de plantes, de cellules végétales, de tissus végétaux ou de parties de plantes.

11. Utilisation selon la revendication 10, **caractérisée en ce que** la plante transformée est choisie parmi des plantes cultivées, telles que des céréales, le maïs, le soja, le riz, le coton, la betterave à sucre, le colza à faible teneur en acide érucique, le tournesol, le lin, la pomme de terre, le tabac, la tomate, le colza, la luzerne, les salades et les différentes espèces d'arbres, de fruitiers à coque et de vigne.

12. Utilisation d'un promoteur selon l'une quelconque des revendications 1 à 4,
a) pour la production d'un bioréacteur végétal ;
b) pour la modification de la composition de composants de plantes ;
c) pour la manipulation du métabolisme végétal ; et
d) pour le transfert de gènes de résistance à des plantes.

13. Plante transgénique, transformée par un vecteur selon la revendication 7 ou par un micro-organisme selon la revendication 8 ou 9, ou cellules transgéniques, tissus transgéniques, parties transgéniques ou matériel de multiplication transgénique d'une telle plante.

14. Plante transgénique selon la revendication 13, choisie parmi des plantes cultivées, telles que des céréales, le maïs, le soja, le riz, le coton, la betterave à sucre, le colza à faible teneur en acide érucique, le tournesol, le lin, la pomme de terre, le tabac, la tomate, le colza, la luzerne, les salades et les différentes espèces d'arbres, de fruitiers à coque et de vigne.

15. Procédé pour la production de plantes transgéniques selon la revendication 13 ou 14, **caractérisé en ce qu'**on transforme des cellules végétales, des tissus végétaux ou des parties de plantes ou des protoplastes par un vecteur selon la revendication 7 ou par un micro-organisme selon la revendication 8 ou 9, on cultive dans un milieu de croissance les cellules, tissus, parties de plantes ou protoplastes transformés et éventuellement on régénère des plantes à partir de la culture.

16. Procédé pour l'isolement d'un promoteur spécifique de mésophylle selon la revendication 1, **caractérisé en ce que**
a) on effectue l'hybridation d'une banque génomique végétale avec de l'ADNc de cytFBPase, n° EMBL X76946,
b) on isole des clones et
c) on teste les clones isolés quant à l'activité du promoteur.

17. Séquence d'acide nucléique choisie parmi SEQ ID n° 1, 2, 3, 4 et 5.

18. Equivalent fonctionnel d'un promoteur selon l'une quelconque des revendications 1 à 4, qui provoque chez des plantes une expression, spécifique du mésophylle, d'une séquence de nucléotides codante, sous le contrôle d'un tel promoteur, l'équivalent fonctionnel étant choisi parmi
a) des variants, apparaissant dans la nature, des séquences selon SEQ ID n° 1 à 5 ;
b) des séquences artificielles dérivées des séquences selon SEQ ID n° 1 à 5, adaptées à l'utilisation de codons d'une plante ;
c) des mutants des séquences selon SEQ ID n° 1 à 5, obtenus par remplacement, addition, délétion, échange ou insertion d'un ou plusieurs résidus nucléotidiques selon SEQ ID n° 1 à 5, qui comportent au moins l'une des séquences répétées suivantes, d'une longueur d'au moins 10 pb :
| Séquence répétée 5' 3' |
|---|
| AAGGATATTT |
| TCTTTTTTTTT |
| ATATGTGACGTGG |
| ATAGAAACAAA |
| GTGCCAACCACT |
| CTCTTTCCACGTG |
| Séquence répétée 5' 3' |
|---|
| CAAACATTTT |
| TTTTTAAAAA |
| AACTTCTGTT |
| TGCATATGCA |
| TGCAGCTGCA |
| TGTATATCAAA |
| TCATCCAAAC |
| TTTTATAAAA |
| TCTGACGTCAGA |
